# FASCICULE DE BREVET EUROPEEN

(11) **EP 1 738 800 B1**
(45) Date de publication et mention de la délivrance du brevet: **09.06.2010**
(21) Numéro de dépôt: 06116140.2
(22) Date de dépôt: 27.06.2006
(51) Int. Cl.: A61Q 5/10

(54) **Composition pour la décoloration et la Coloration simultanées des fibres kératiniques comprenant un colorant anionique ou non ionique et un polymère associatif**
Mittel zum gleichteitigen Bleichen oder Färben von Keratinfasern enthaltend einen anionischen oder nichtionischen Farbstoff und ein assoziatives Polymer
Agent for simultaneously bleaching and coloring of keratin fibres comprising an anionic or non-ionic dye and an asociative polymer

(30) Priorité: 29.06.2005 FR 0551812
(43) Date de publication de la demande: 03.01.2007
(73) Titulaire: L'Oréal, 75008 Paris (FR)
(72) Inventeur: Kravtchenko, Sylvain, At Xintiandi 200021, Shanghaï (CN)
(74) Mandataire: Prevel, Estelle Nicole

(56) Documents cités:
- WO-A-01/28508
- WO-A-02/074270
- WO-A-2004/078150
- GB-A- 859 550
- US-A- 3 578 387
- US-A1- 2002 004 957

## Description

La présente invention a pour objet une composition pour la décoloration et la coloration simultanées des fibres kératiniques, et en particulier des fibres kératiniques humaines telles que les cheveux.

Lorsqu'une personne souhaite changer radicalement de couleur de cheveux, notamment lorsqu'elle souhaite obtenir une couleur plus claire que sa couleur d'origine, il est souvent nécessaire de procéder à une décoloration, et éventuellement à une coloration des cheveux. Pour ce faire, il existe plusieurs méthodes.

La première méthode consiste à utiliser des produits éclaircissants à base d'ammoniaque et de peroxyde d'hydrogène. Ces produits peuvent éventuellement contenir des colorants ce qui permet d'éclaircir et de colorer simultanément les cheveux. Toutefois, les performances éclaircissantes de ces produits restent limitées, plus particulièrement pour des applications sur des cheveux à fonds foncés naturels et / ou colorés.

La deuxième méthode consiste à appliquer sur les cheveux une composition éclaircissante à base de sels peroxygénés tels que les persulfates et d'agents alcalins dans laquelle a été ajouté du peroxyde d'hydrogène au moment de l'emploi, afin d'obtenir un éclaircissement plus important. Ce type de produit est très satisfaisant et plus adapté à des fonds foncés, mais il ne conduit qu'à une gamme très restreinte de reflets. Il est alors nécessaire de corriger la nuance obtenue en appliquant dans un deuxième temps un produit de coloration sur les cheveux. Ce procédé en deux étapes présente l'inconvénient d'être relativement long.

Pour pallier à cet inconvénient, il a été proposé, dans le brevet US 5 688 291, la demande de brevet WO 02/074270 et le modèle d'utilité DE 203 03 559, d'ajouter à ces produits éclaircissants des colorants, et en particulier des colorants directs de type anthraquinone, azo, triarylméthane, thiazine, quinone et nitro, qui sont pour certains stables dans ces milieux fortement oxydants. Cette méthode permet de colorer et de décolorer simultanément la fibre capillaire. Le niveau d'éclaircissement étant important, elle est particulièrement bien adaptée à des fonds foncés naturels et / ou colorés. Cependant, ces produits ont l'inconvénient de se présenter sous forme de poudres qui sont volatiles et de praticité réduite.

Le but de la présente invention est de fournir de nouvelles compositions pour la décoloration et la coloration simultanées des fibres kératiniques, et en particulier des fibres kératiniques humaines telles que les cheveux, particulièrement bien adaptées à des fonds foncés, qui sont faciles à utiliser et qui permettent d'obtenir des colorations chromatiques et tenaces.

Ce but est atteint avec la présente invention qui a pour objet une composition pour la décoloration et la coloration simultanées des fibres kératiniques comprenant :
- au moins un colorant direct choisi parmi les colorants anioniques et non ioniques, à l'exception du 7-(6'-méthylphénylazo)-1-acétamido-3,6-disulfo-8-hydroxy-naphtalène, des ortho nitro-anilines substituées en méta du groupement amino, de la quinoline, des dérivés quinoliniques, et de leurs sels d'addition ;
- au moins un polymère associatif ;
- au moins un sel peroxygéné ; et
- au moins un agent alcalin.

La composition conforme à la présente invention est particulièrement bien adaptée pour la décoloration et la coloration simultanées des cheveux foncés. Elle est facile à utiliser et permet d'obtenir une coloration chromatique. De plus, avec des concentrations adaptées en colorants selon l'invention, on peut obtenir une large palette de couleurs et de reflets.

Cette coloration est résistante aux diverses agressions que peuvent subir les cheveux telles que les shampoings, les frottements, la lumière, les intempéries, la sueur et les déformations permanentes. Elle est aussi puissante, esthétique, et de plus peu sélective, c'est-à-dire qu'elle permet d'obtenir de faibles écarts entre différentes parties différemment sensibilisées d'un cheveu ou d'une chevelure.

Par ailleurs, lorsque la composition de l'invention se présente sous forme de poudre, le mélange avec une composition aqueuse contenant du peroxyde d'hydrogène est plus facile à réaliser que dans le cas des poudres classiques.

La présente invention a également pour objet un procédé de décoloration et de coloration simultanées des fibres kératiniques mettant en oeuvre la composition conforme à l'invention, ainsi que des dispositifs à plusieurs compartiments pour la mise en oeuvre de ce procédé.

Un autre objet de la présente invention est l'utilisation de la composition conforme à l'invention pour la décoloration et la coloration simultanées des fibres kératiniques.

Les colorants anioniques utiles dans le cadre de l'invention peuvent être choisis parmi les colorants directs nitrés acides, les colorants azoïques acides, les colorants aziniques acides, les colorants triarylméthaniques acides, les colorants indoaminiques acides, les colorants naturels acides.

De préférence, les colorants anioniques utiles dans le cadre de l'invention sont choisis parmi les composés suivants :

| | |
|---|---|
| (C.I. 45380) | Acid Red 87 |
| (C.I. 10316) | Sel de sodium de l'acide 2,4-dinitro-1-naphtol-7-sulfonique |
| (C.I. 10383) | Acid Orange 3 |
| (C.I. 13015) | Acid Yellow 9 / Food Yellow 2 |
| (C.I. 14780) | Direct Red 45 / Food Red 13 |
| (C.I. 13711) | Acid Black 52 |
| (C.I. 13065) | Acid Yellow 36 |
| (C.I. 14700) | Sel de sodium de l'acide 1-hydroxy-2-(2',4'-xylyl-5-sufonatoazo)-naphtalène-4-sulfonique / Food Red 1 |
| (C.I. 14720) | Acid Red 14 / Food Red 3 / Mordant Blue 79 |
| (C. I. 14805) | Acid Brown 4 |
| (C.I. 15510) | Acid Orange 7 / Pigment Orange 17 / Solvent Orange 49 |
| (C.I. 15985) | Food Yellow 3 / Pigment Yellow 104 |
| (C.I. 16185) | Acid Red 27 / Food Red 9 |
| (C.I. 16230) | Acid Orange 10 / Food Orange 4 |
| (C.I. 16250) | Acid Red 44 |
| (C.I. 17200) | Acid Red 33 / Food Red 12 |
| (C.I. 15685) | Acid Red 184 |
| (C.I. 19125) | Acid Violet 3 |
| (C.I. 18055) | Sel de sodium de l'acide 1-hydroxy-2-(4'-acétamido phénylazo)-8-acétamido-naphtalène-3,6-disulfonique / Acid Violet 7 / Food Red 11 |
| (C.I. 18130) | Acid Red 135 |
| (C.I. 19130) | Acid Yellow 27 |
| (C.I. 19140) | Acid Yellow 23 / Food Yellow 4 |
| (C.I. 20170) | 4'-(sulfonato-2",4"-diméthyl)-bis-(2,6-phénylazo)-1,3-dihydroxy benzène / Acid Orange 24 |
| (C.I. 20470) | Sel de sodium de l'acide 1-amino-2-(4'-nitrophénylazo)-7-phénylazo-8-hydroxy-naphtalène-3,6-disulfonique / Acid Black 1 |
| (C.I. 23266) | (4-((4-méthylphényl) sulfonyloxy)-phénylazo)2,2'-diméthyl-4-((2-hydroxy-5,8-disulfonato)naphtylazo)biphényle / Acid Red 111 |
| (C.I. 27755) | Food Black 2 |
| (C.I. 25440) | 1-(4'-sulfonatophénylazo)-4-((2"-hydroxy-3"-acétylamino-6",8"-disulfonato)naphtylazo)-6-sulfonatonaphtalène (sel tétrasodique) / Food Black 1 |
| (C.I. 42080) | Acide 4-β-hydroxyéthylamino-3-nitrobenzènesulfonique |
| (C.I. 42090) | Acid Blue 9 |
| (C.I. 60730) | Acid Violet 43 |
| (C.I. 61570) | Acid Green 25 |
| (C.I. 62045) | Sel de sodium de l'acide 1-amino-4-cyclohexylamino-9,10-anthraquinone 2-sulfonique / Acid Blue 62 |
| (C.I. 62105) | Acid Blue 78 |
| (C.I. 14710) | Sel de sodium de l'acide 4-hydroxy-3((2-méthoxy phényl)-azo)-1-naphtalène sulfonique / Acid Red 4 |
| | Acide 2-pipéridino 5-nitro benzène sulfonique |
| | Acide 2(4'-N,N(2"-hydroxyéthyl)amino-2'-nitro)aniline éthane sulfonique |
| | Acide 4-β-hydroxyéthylamino-3-nitrobenzène sulfonique |
| (C.I. 42640) | Acid Violet 49 |
| (C.I. 42080) | Acid Blue 7 |
| - | Acid Blue 156 |
| - | Acid Blue 317 |
| (C.I. 58005) | Sel de sodium du 1,2-dihydroxy-3-sulfo-anthraquinone / Mordant Red 3 |
| (C.I. 62055) | Sel de sodium de l'acide 1-amino-9,10-dihydro-9,10-dioxo-4-(phénylamino) 2-anthracène sulfonique / Acid Blue 25 |
| (C.I. 14710) | Sel de sodium de l'acide 4-hydroxy-3-((2-méthoxyphényl)-azo)-1-naphtalène sulfonique / Acid Red 4 |

La plupart de ces colorants sont décrits en particulier dans le Color Index publié par The Society of Dyers and Colorists, P.O. Box 244, Perkin House, 82 Grattan Road, Bradford, Yorkshire, BD1 2JBN England.

Les colorants anioniques plus particulièrement préférés sont les colorants désignés dans le Color Index sous le code C.I. 58005 (sel monosodique de l'acide 1,2-dihydroxy-9,10-anthraquinone-3-sulfonique), C.I. 60730 (sel monosodique de l'acide 2-[(9,10-dihydro-4-hydroxy-9,10-dioxo-1-anthracényl)-amino]-5-méthyl-benzène sulfonique), C.I. 15510 (sel monosodique de l'acide 4-[(2-hydroxy-1-naphtalényl)-azo]-benzène sulfonique), C.I. 15985 (sel disodique de l'acide 6-hydroxy-5-[(4-sulfophényl)-azo]-2-naphtalène sulfonique), C.I. 17200 (sel disodique de l'acide 5-amino-4-hydroxy-3-(phénylazo)-2,7-naphtalène disulfonique), C.I. 20470 (sel disodique de l'acide 1-amino-2-(4'-nitrophénylazo)-7-phénylazo-8-hydroxy-3,6-naphtalène disulfonique), C.I. 42090 (sel disodique du N-éthyl-N-[4-[[4-[éthyl[3-sulfophényl)-méthyl]-amino]-phényl](2-sulfophényl)-méthylène]-2,5-cyclohexadien-1-ylidène]-3-sulfobenzenemethanaminium hydroxyde, sel interne), C.I. 61570 ( sel disodique de l'acide 2,2'-[(9,10-dihydro-9,10-dioxo-1,4-anthracènediyl)-dümino]-bis-[5-méthyl]-benzène sulfonique.

Le ou les colorants anioniques pouvant être utilisés dans le cadre de la présente invention sont également préférentiellement choisis parmi les composés suivants :
- l'acide 4-(β-hydroxyéthyl)amino-3-nitro-benzènesulfonique,
- l'acide 4-N-éthylamino-3-nitrobenzoique ;
- l'acide 2-pipéridino-5-nitrobenzoique ;
- l'acide 4-amino-2-nitrodiphénylamine-2' carboxylique ;
- l'acide 4-amino-4'-diméthylamino-2-nitrodiphénylamine-2'-carboxylique ;
- l'acide 3-oxo-6-hydroxy-9-carboxy-phényl xanthylium.

Le ou les colorants non ioniques utiles dans le cadre de l'invention peuvent être choisis parmi les colorants non ioniques benzéniques nitrés, les colorants non ioniques azoïques, azométhiniques, méthiniques, anthraquinoniques, naphtoquinoniques, benzoquinoniques, phénotiaziniques indigoïdes, xanthéniques, phénanthridiniques, phtalocyanines, ceux dérivés du triarylméthane, seuls ou en mélanges.

Ils peuvent par exemple être choisis parmi les colorants benzéniques nitrés rouges ou orangés, par exemple les composés suivants :
- le 1-hydroxy-3-nitro-4-N-(γ-hydroxypropyl)amino benzène,
- le N-(β-hydroxyéthyl)amino-3-nitro-4-amino benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- la 2-nitro-4-amino-diphénylamine,
- le 1-amino-3-nitro-6-hydroxybenzène.
- le 1-(β-aminoéthyl)amino-2-nitro-4-(β-hydroxyéthyloxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine.

Ils peuvent également être choisis parmi les colorants directs benzéniques nitrés jaunes et jaune-verts, tels que les composés suivants :
- le 1-(β-hydroxyéthyl)amino-2-méthoxy-4-nitrobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- le 4-(β-hydroxyéthyl)amino-3-nitro-chlorobenzène,
- le 4-(β-hydroxyéthyl)amino-3-nitro-méthylbenzène,
- le 4-(β,γ -dihydroxypropyl)amino-3-nitro-trifluorométhylbenzène,
- le 1-(β-uréidoéthyl)amino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

Ils peuvent aussi être choisis parmi les colorants directs benzéniques nitrés bleus ou violets. On peut par exemple citer les composés suivants :
- le 1-(β-hydroxyéthyl)amino-4-N,N-bis-(β-hydroxyéthyl)amino 2-nitrobenzène,
- le 1-(y-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 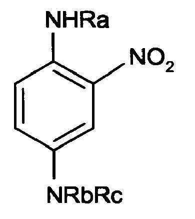
dans laquelle :
- Rb représente un radical alkyle en C₁-C₄, un radical β-hydroxyéthyle ou β-hydroxypropyle ou γ-hydroxypropyle;
- Ra et Rc, identiques ou différents, représentent un radical β-hydroxyéthyle, -β-hydroxypropyle, γ-hydroxypropyle, ou β,γ-dihydroxypropyle, l'un au moins des radicaux Rb, Rc ou Ra représentant un radical γ-hydroxypropyle et Rb et Rc ne pouvant désigner simultanément un radical β-hydroxyéthyle lorsque Ra est un radical γ-hydroxypropyle, telles que ceux décrits dans le brevet français FR 2 692 572.

A titre de colorants non ioniques, on peut aussi citer les colorants suivants: le Disperse Orange 3 ; le Disperse Red 17 ; le Disperse Violet 4 ; le Disperse Violet 8 ; le Disperse Blue 1 ; le Disperse Red 15 ; le Solvent Violet 13 ; le Solvent Violet 11 ; le Disperse Blue 3 ; le Disperse Blue 7 ; le Disperse Red 11 ; le Natural Brown 7 ; le Disperse Black 9 ; le Disperse Violet 15 ; le Natural Orange 6 ; la 2-hydroxy 3-méthoxy 1,4-naphtoquinone ; la 3-hydroxy 2-méthyl 1,4-naphtoquinone ou le phtiocol ; la 3,6-dihydroxy 5-méthoxy p-toluquinone ou la spinulosine ; le HC Blue 14.

De préférence, le ou les colorants non ioniques sont choisis parmi le 1-hydroxy-3-nitro-4-N-(γ-hydroxypropyl)amino benzène; le N-(β-hydroxyéthyl)amino-3-nitro-4-amino benzène ; le 1-hydroxy-3-nitro-4-N-(β-hydroxyéthyl)amino benzène ; le 1,4-diamino-2-nitrobenzène ; le 1-amino-2-nitro-4-méthylamino benzène ; la N-(β-hydroxyéthyl)-2-nitro-paraphénylènediamine ; la 2-nitro-4-amino-diphénylamine ; le 1-amino-3-nitro-6-hydroxybenzène ; le 1-(β-aminoéthyi)amino-2-nitro-4-(β-hydroxyéthyloxy) benzène ; le 1-(β, γ-dihydroxypropyl)oxy-3-nitro-4-(β-hydroxyéthyl)amino benzène ; le 1-hydroxy-3-nitro-4-aminobenzène ; le 1-hydroxy-2-amino-4,6-dinitrobenzène ; le 1-méthoxy-3-nitro-4-(β-hydroxyéthyl)amino benzène ; la 2-nitro-4'-hydroxydiphénylamine ; le 1-(β-hydroxyéthyl)amino-2-méthoxy-4-nitrobenzène ; le 1-amino-2-nitro-6-méthyl-benzène ; le 1-(β-hydroxyéthyi)amino-2-hydroxy-4-nitrobenzène ; la N-(β-hydroxyéthyl)-2-nitro-4-trifluorométhylaniline ; le 4-(β-hydroxyéthyl)amino-3-nitro-chlorobenzène ; le 4-(β-hydroxyéthyi)amino-3-nitro-méthylbenzène ; le 4-(β,γ -dihydroxypropyl)amino-3-nitro-trifluorométhylbenzène ; le 1-(β-uréidoéthyl)amino-4-nitrobenzène ; le 1-hydroxy-2-amino-5-nitrobenzène ; le 1-amino-2-[tris(hydroxyméthyl)méthyl]amino-5-nitro-benzène ; le 1-(β-hydroxyéthyl)amino-2-nitrobenzène ; le 4-(β-hydroxyéthyl)amino-3-nitrobenzamide ; le 1-(β-hydroxyéthyl)amino-4-N,N-bis-(β-hydroxyéthyl)amino 2-nitrobenzène ; le 1-(y-hydroxypropyl)amino 4-N,N-bis-(β-hydroxyéthyl)amino 2-nitrobenzène ; le 1-(β-hydroxyéthyi)amino 4-(N-méthyl, N-β-hydroxyéthyl)amino 2-nitrobenzène ; le 1-(β-hydroxyéthyl)amino 4-(N-éthyl, N-β-hydroxyéthyl)amino 2-nitrobenzène ; le 1-(β,γ-dihydroxypropyl)amino 4-(N-éthyl, N-β-hydroxyéthyl)amino 2-nitrobenzène ; le Disperse Orange 3 ; le Disperse Red 17 ; le Disperse Violet 4 ; le Disperse Violet 8 ; le Disperse Blue 1 ; le Disperse Red 15 ; le Solvent Violet 13 ; le Solvent Violet 11 ; le Disperse Blue 3 ; le Disperse Blue 7 ; le Disperse Red 11 ; le Natural Brown 7 ; le Disperse Black 9 ; le Disperse Violet 15 ; le Natural Orange 6; la 2-hydroxy 3-méthoxy 1,4-naphtoquinone ; la 3-hydroxy 2-méthyl 1,4-naphtoquinone ou le phtiocol ; la 3,6-dihydroxy 5-méthoxy p-toluquinone ou la spinulosine ; le HC Blue 14.

La concentration en colorants anioniques et / ou non ioniques dans la composition conforme à l'invention est généralement comprise entre 0,0001 et 10 % en poids, de préférence entre 0,001 et 8 %, et encore plus préféntiellement entre 0,01 et 5 % en poids du poids total de la composition.

Les polymères associatifs sont des polymères hydrosolubles capables, dans un milieu aqueux, de s'associer réversiblement entre eux ou avec d'autres molécules. Leur structure chimique comprend des zones hydrophiles, et des zones hydrophobes caractérisées par au moins une chaîne grasse.

Les polymères associatifs selon l'invention peuvent être de type anionique, cationique, amphotère ou non ionique. De préférence, les polymères associatifs sont cationiques ou non ioniques.

Parmi les polymères associatifs de type anionique, on peut citer :
- (I) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux-ci, et dont le motif éther d'allyle à chaîne grasse correspond au monomère de formule (I) suivante :

   CH₂ = C R' CH₂ O Bₙ R (I)
dans laquelle R' désigne H ou CH₃, B désigne le radical éthylèneoxy, n est nul ou désigne un entier allant de 1 à 100, R désigne un radical hydrocarboné choisi parmi les radicaux alkyle, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryl (C₁₈).

Des polymères associatifs anioniques de ce type sont décrits et préparés, selon un procédé de polymérisation en émulsion, dans le brevet EP-0 216 479.

Parmi ces polymères associatifs anioniques, on préfère particulièrement selon l'invention les polymères formés à partir de 20 à 60 % en poids d'acide acrylique et / ou d'acide méthacrylique, de 5 à 60 % en poids de (méth)acrylates d'alkyles inférieurs, de 2 à 50 % en poids d'éther d'allyle à chaîne grasse de formule (I), et de 0 à 1 % en poids d'un agent réticulant qui est un monomère insaturé polyéthylénique copolymérisable bien connu, comme le phtalate de diallyle, le (méth)acrylate d'allyle, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80® et SALCARE SC90® qui sont des émulsions aqueuses à 30 % d'un terpolymère réticulé d'acide méthacrylique, d'acrylate d'éthyle et de steareth-10-allyl éther (40 / 50 /10).
- (II) ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (II) suivante : 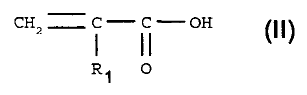
dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (III) suivante : 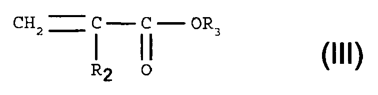 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.

Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères anioniques de ce type sont par exemple décrits et préparés, selon les brevets US-3 915 921 et 4 509 949.

Parmi ce type de polymères associatifs anioniques, on utilisera plus particulièrement des polymères formés à partir d'un mélange de monomères comprenant :
(i) essentiellement de l'acide acrylique,
(ii) un ester de formule (III) décrite ci-dessus et dans laquelle R₂ désigne H ou CH₃, R₃ désignant un radical alkyle ayant de 12 à 22 atomes de carbone,
(iii) et un agent réticulant, qui est un monomère insaturé polyéthylénique copolymérisable bien connu, comme le phtalate de diallyle, le (méth)acrylate d'allyle, le divinylbenzène, le diméthacrylate de (poly)éthylèneglycol, et le méthylène-bis-acrylamide.

Parmi ce type de polymères associatifs anioniques, on utilisera plus particulièrement ceux constitués de 95 à 60 % en poids d'acide acrylique (motif hydrophile), 4 à 40 % en poids d'acrylate d'alkyles en C₁₀-C₃₀ (motif hydrophobe), et 0 à 6 % en poids de monomère polymérisable réticulant, ou bien ceux constitués de 98 à 96 % en poids d'acide acrylique (motif hydrophile), 1 à 4 % en poids d'acrylate d'alkyles en C₁₀-C₃₀ (motif hydrophobe), et 0,1 à 0,6 % en poids de monomère polymérisable réticulant tel que ceux décrits précedemment.

Parmi lesdits polymères ci-dessus, on préfère tout particulièrement selon la présente invention, les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.
- (III) les terpolymères d'anhydride maléique / α-oléfine en C₃₀-C₃₈ /maléate d'alkyle tel que le produit (copolymère anhydride maléique / α-oléfine en C₃₀-C₃₈ / maléate d'isopropyle) vendu sous le nom PERFORMA V 1608® par la société NEWPHASE TECHNOLOGIES.
- (IV) les terpolymères acryliques comprenant :
   (a) environ 20 % à 70 % en poids d'un acide carboxylique à insaturation α,β-monoéthylénique,
   (b) environ 20 à 80 % en poids d'un monomère à insaturation α,β-monoéthylénique non-tensio-actif différent de (a),
   (c) environ 0,5 à 60 % en poids d'un mono-uréthane non-ionique qui est le produit de réaction d'un tensio-actif monohydrique avec un monoisocyanate à insaturation monoéthylénique,
      tels que ceux décrits dans la demande de brevet EP-A-0173109 et plus particulièrement celui décrit dans l'exemple 3, à savoir, un terpolymère acide méthacrylique / acrylate de méthyle / diméthyl métaisopropényl benzyl isocyanate d'alcool béhényle éthoxylé (40 OE) en dispersion aqueuse à 25 %.
- (V) les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C₁-C₄.

A titre d'exemple de ce type de composé on peut citer l'ACULYN 22® vendu par la société ROHM et HAAS, qui est un terpolymère acide méthacrylique / acrylate d'éthyle / méthacrylate de stéaryle oxyalkyléné.

Parmi les polymères associatifs de type cationique, on peut citer :
- (I) les polyuréthanes associatifs cationiques dont la famille a été décrite par la demanderesse dans la demande de brevet français N°-0009609 ; elle peut être représentée par la formule générale (IV) suivante :

   R-X-(P)ₙ-[L-(Y)ₘ]ᵣ-L'-(P')ₚ-X'-R' (IV)
dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000;
la molécule contenant au moins une fonction amine protonée ou quaternisée et au moins un groupement hydrophobe.

Dans un mode de réalisation préféré de ces polyuréthanes, les seuls groupements hydrophobes sont les groupes R et R' aux extrémités de chaîne.

Une famille préférée de polyuréthanes associatifs cationiques est celle correspondant à la formule (IV) décrite ci-dessus dans laquelle :
R et R' représentent tous les deux indépendamment un groupement hydrophobe,
X, X' représentent chacun un groupe L",
n et p valent entre 1 et 1000, et
L, L', L", P, P', Y et m ont la signification indiquée ci-dessus.

Une autre famille préférée de polyuréthanes associatifs cationiques est celle correspondant à la formule (IV) ci-dessus dans laquelle :
R et R' représentent tous les deux indépendamment un groupement hydrophobe, X, X' représentent chacun un groupe L", n et p valent 0, et L, L', L", Y et m ont la signification indiquée ci-dessus.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate, etc.

Encore une autre famille préférée de polyuréthanes associatifs cationiques est celle correspondant à la formule (IV) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.

Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel.

A titre d'exemple, le groupement hydrophobe peut être issu d'un alcool gras tel que l'alcool stéarylique, l'alcool dodécylique, l'alcool décylique. II peut également désigner un polymère hydrocarboné tel que par exemple le polybutadiène.

Lorsque X et / ou X' désignent un groupement comportant une amine tertiaire ou quaternaire, X et / ou X' peuvent représenter l'une des formules suivantes : 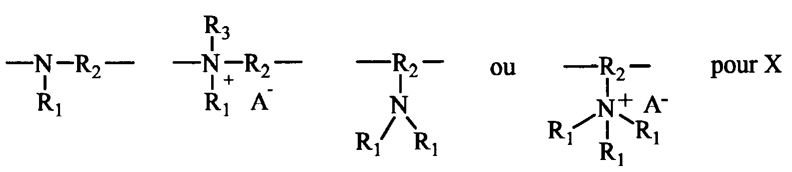 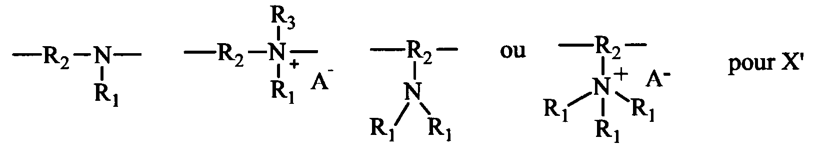 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacés par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacés par un hétéroatome choisi parmi N, S, O, P ;
A⁻ est un contre-ion physiologiquement acceptable.

Les groupements L, L' et L" représentent un groupe de formule : 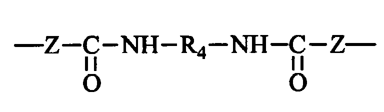 dans laquelle :
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacés par un hétéroatome choisi parmi N, S, O et P.

Les groupements P et P', comprenant une fonction amine peuvent représenter au moins l'une des formules suivantes : 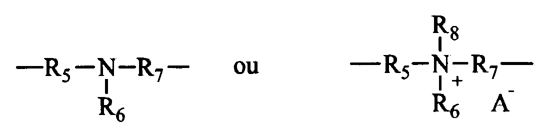 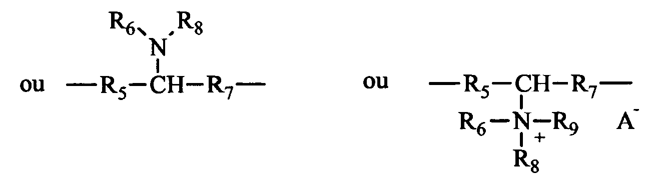 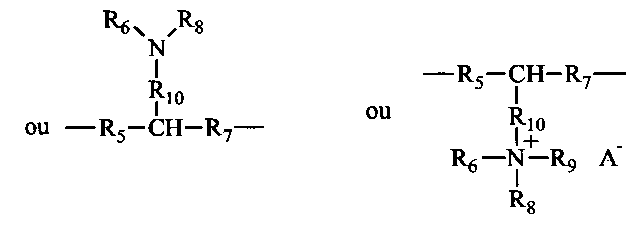 dans lesquelles :
R₅ et R₇ ont les mêmes significations que R₂ défini précédemment ;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P,
et A⁻ est un contre-ion physiologiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (IV) selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (IV) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.

Comme indiqué précédemment, ce composé peut comporter plus d'un motif à fonction amine. Il s'agit alors d'un polymère portant une répétition du motif à fonction amine.

Ce type de composés peut être représenté par l'une des formules suivantes :

HZ-(P)ₙ-ZH,

ou

HZ-(P')p-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (IV) est un diisocyanate correspondant à la formule :

O=C=N-R₄-N=C=O

dans laquelle R₄ est défini plus haut.

A titre d'exemple, on peut citer le méthylènediphényldiisocyanate, le méthyiènecyciohexanediisocyanate, l'isophoronediisocyanate, le toluènediisocyanate, le naphtalènediisocyanate, le butanediisocyanate, l'hexanediisocyanate.

Un troisième composé entrant dans la préparation du polyuréthane de formule (IV) est un composé hydrophobe destiné à former les groupes hydrophobes terminaux du polymère de formule (IV).

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné α-hydroxyle.

Le groupe hydrophobe du polyuréthane de formule (IV) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate, etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemple, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (IV) est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthanes associatifs cationiques comportant un tel groupe.
- (II) les dérivés de cellulose quaternisée et les polyacrylates à groupements latéraux aminés non cycliques.

Les dérivés de cellulose quaternisée sont en particulier :
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthylcelluloses quaternisées à chaînes grasses en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C₁₂) et CRODACEL QS® (alkyle en C₁₈) commercialisés par la société CRODA. (III)- Les polyvinyllactames cationiques dont la famille a été décrite par la demanderesse dans la demande de brevet français N°-0101106.

Lesdits polymères comprennent :
-a) au moins un monomère de type vinyl lactame ou alkylvinyllactame,
-b) au moins un monomère de structures (V) ou (VI) suivantes : 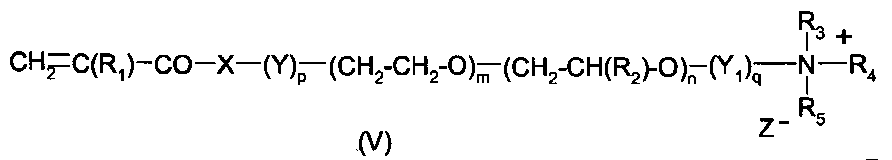 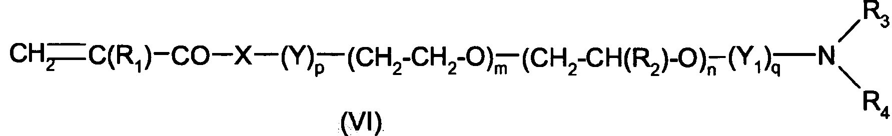
dans lesquelles :
X désigne un atome d'oxygène ou un radical NR₆,
R₁ et R₆ désignent, indépendamment l'un de l'autre, un atome d'hydrogène ou un radical alkyl linéaire ou ramifié en C₁-C₅,
R₂ désigne un radical alkyle linéaire ou ramifié en C₁-C₄,
R₃, R₄ et R₅ désignent, indépendamment l'un de l'autre, un atome d'hydrogène, un radical alkyle linéaire ou ramifié en C₁-C₃₀ ou un radical de formule (VII) : 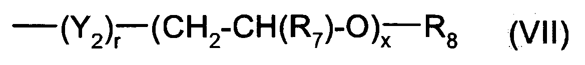
Y , Y₁ et Y₂ désignent, indépendamment l'un de l'autre, un radical alkylène linéaire ou ramifié en C₂-C₁₆,
R₇ désigne un atome d'hydrogène, ou un radical alkyle linéaire ou ramifié en C₁-C₄ ou un radical hydroxyalkyle linéaire ou ramifié en C₁-C₄,
R₈ désigne un atome d'hydrogène ou un radical alkyle linéaire ou ramifié en C₁-C₃₀,
p, q et r désignent, indépendamment l'un de l'autre, soit la valeur zéro, soit la valeur 1,
m et n désignent, indépendamment l'un de l'autre, un nombre entier allant de 0 à 100,
x désigne un nombre entier allant de 1 à 100,
Z désigne un anion d'acide organique ou minéral,
sous réserve que :
- l'un au moins des substituants R₃, R₄ R₅ ou R₈ désigne un radical alkyle linéaire ou ramifié en C₉-C₃₀,
- si m ou n est différent de zéro, alors q est égal à 1,
- si m ou n sont égaux à zéro, alors p ou q est égal à 0.

Les polymères poly(vinyllactame) cationiques selon l'invention peuvent être réticulés ou non réticulés et peuvent aussi être des polymères blocs.

De préférence le contre ion Z- des monomères de formule (V) est choisi parmi les ions halogénures, les ions phosphates, l'ion méthosulfate, l'ion tosylate.

De préférence R₃, R₄ et R₅ désignent, indépendamment l'un de l'autre, un atome d'hydrogène ou un radical alkyle linéaire ou ramifié en C₁-C₃₀.

Plus préférentiellement, le monomère b) est un monomère de formule (V) pour laquelle, encore plus préférentiellement, m et n sont égaux à zéro.

Le monomère vinyl lactame ou alkylvinyllactame est de préférence un composé de structure (VIII) : 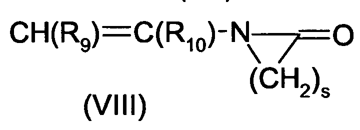 dans laquelle :
s désigne un nombre entier allant de 3 à 6,
R₉ désigne un atome d'hydrogène ou un radical alkyle en C₁-C₅,
R₁₀ désigne un atome d'hydrogène ou un radical alkyle en C₁-C₅,
sous réserve que l'un au moins des radicaux R₉ et R₁₀ désigne un atome d'hydrogène.

Encore plus préférentiellement, le monomère (VIII) est la vinylpyrrolidone.

Les polymères poly(vinyllactame) cationiques selon l'invention peuvent également contenir un ou plusieurs monomères supplémentaires, de préférence cationiques ou non ioniques.

A titre de composés plus particulièrement préférés selon l'invention, on peut citer les terpolymères suivants comprenant au moins :
a)-un monomère de formule (VIII),
b)-un monomère de formule (V) dans laquelle p = 1, q = 0, R₃ et R₄ désignent, indépendamment l'un de l'autre, un atome d'hydrogène ou un radical alkyle en C₁-C₅ et R₅ désigne un radical alkyle en C₉-C₂₄ et
c)-un monomère de formule (VI) dans laquelle R₃ et R₄ désignent, indépendamment l'un de l'autre, un atome d'hydrogène ou un radical alkyle en C₁-C₅.

Encore plus préférentiellement, on utilisera les terpolymères comprenant, en poids, 40 à 95 % de monomère (a), 0,1 à 55 % de monomère (c) et 0,25 à 50 % de monomère (b).

De tels polymères sont décrits dans la demande de brevet WO-00/68282 dont le contenu fait partie intégrante de l'invention.

Comme polymères poly(vinyllactame) cationiques selon l'invention, on utilise notamment les terpolymères vinylpyrrolidone /diméthylaminopropylméthacrylamide / tosylate de dodécyldiméthylméthacrylamidopropylammonium, les terpolymères vinylpyrrolidone / diméthylaminopropylméthacrylamide / tosylate de cocoyldiméthylméthacrylamido propylammonium, les terpolymères vinylpyrrolidone / diméthylaminopropyl méthacrylamide / tosylate ou chlorure de lauryldiméthylméthacrylamidopropylammonium.

La masse moléculaire en poids des polymères poly(vinyllactame) cationiques selon la présente invention est de préférence comprise entre 500 et 20 000 000. Elle est plus particulièrement comprise entre 200 000 et 2 000 000 et encore plus préférentiellement comprise entre 400 000 et 800 000.

Les polymères associatifs amphotères sont choisis de préférence parmi ceux comportant au moins un motif cationique non cyclique. Plus particulièrement encore, on préfère ceux préparés à partir ou comprenant 1 à 20 moles % de monomère comportant une chaîne grasse, et de préférence 1,5 à 15 moles % et plus particulièrement encore 1,5 à 6 moles %, par rapport au nombre total de moles de monomères.

Les polymères associatifs amphotères préférés selon l'invention comprennent, ou sont préparés en copolymérisant :
1) au moins un monomère de formule (IXa) ou (IXb) : 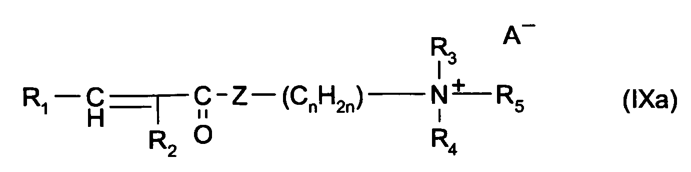 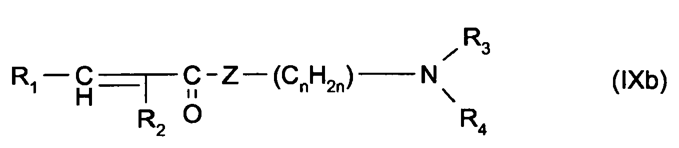 dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A- est un anion issu d'un acide organique ou minéral, tel qu'un anion méthosulfate ou un halogénure tel que chlorure ou bromure ;
2) au moins un monomère de formule (X) :

   R₆―CH= CR₇―COOH (X)

   dans laquelle, R₆ et R₇, identiques ou différents, représentent un atome d'hydrogène ou un radical méthyle ; et
3) au moins un monomère de formule (XI):

   R₆―CH= CR₇― COXR₈ (XI)
dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
l'un au moins des monomères de formule (IXa), (IXb) ou (XI) comportant au moins une chaîne grasse.

Les monomères de formule (IXa) et (IXb) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide, ces monomères étant éventuellement quaternisés, par exemple par un halogénure d'alkyle en C₁-C₄ ou un sulfate de dialkyle en C₁-C₄.

Plus particulièrement, le monomère de formule (IXa) est choisi parmi le chlorure d'acrylamidopropyl triméthyl ammonium et le chlorure de méthacrylamidopropyl triméthyl ammonium.

Les monomères de formule (X) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (X) est l'acide acrylique.

Les monomères de formule (XI) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et / ou quaternisés.

Le rapport du nombre de charges cationiques / charges anioniques est de préférence égal à environ 1.

Les polymères associatifs amphotères selon l'invention comprennent de préférence de 1 à 10 moles % du monomère comportant une chaîne grasse (monomère de formule (IXa), (IXb) ou (XI)), et de préférence de 1,5 à 6 moles %.

Les poids moléculaires moyens en poids des polymères associatifs amphotères selon l'invention peuvent varier de 500 à 50.000.000 et sont de préférence compris entre 10.000 et 5 000 000.

Les polymères associatifs amphotères selon l'invention peuvent également contenir d'autre monomères tels que des monomères non ioniques et en particulier tels que les acrylates ou méthacrylates d'alkyle en C₁-C₄.

Des polymères associatifs amphotères selon l'invention sont par exemple décrits et préparés dans la demande de brevet WO9844012.

Parmi les polymères associatifs amphotères selon l'invention, on préfère les terpolymères acide acrylique / chlorure de (méth)acrylamidopropyl triméthyl ammonium / méthacrylate de stéaryle.

Selon l'invention, les polymères associatifs de type non ioniques sont choisis de préférence parmi :
- (1) les celluloses modifiées par des groupements comportant au moins une chaîne grasse.

On peut citer à titre d'exemple :
- les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
- celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.

- (2) les hydroxypropylguars modifiés par des groupements comportant au moins une chaîne grasse tel que le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.
- (3) les copolymères de vinyl pyrrolidone et de monomères hydrophobes à chaîne grasse dont on peut citer à titre d'exemple :
   - les produits ANTARON V216® ou GANEX V216® (copolymère vinylpyrrolidone / hexadécène) vendu par la société I.S.P.
   - les produits ANTARON V220® ou GANEX V220® (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse tels que par exemple le copolymère acrylate de méthyle / acrylate de stéaryle oxyéthyléné vendu par la société GOLDSCHMIDT sous la dénomination ANTIL 208®.
- (5) les copolymères de méthacrylates ou d'acrylates hydrophiles et de monomères hydrophobes comportant au moins une chaîne grasse tels que par exemple le copolymère méthacrylate de polyéthylèneglycol /méthacrylate de lauryle.
- (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et / ou des enchaînements cycloaliphatiques et / ou aromatiques.
- (7) les polymères à squelette aminoplaste éther possédant au moins une chaîne grasse, tels que les composés PURE THIX® proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse, ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂-C₁₄ et le produit ELFACOS T212® à chaîne alkyle en C₁₈ de chez AKZO.

Le produit DW 1206B® de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple de tels polymères, on peut citer le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4 %) et d'eau (81 %) ; l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35 % en poids dans un mélange de propylèneglycol (39 %) et d'eau (26 %)].

Dans une composition préférée selon la présente invention, les polymères associatifs sont choisis parmi ceux de type non ionique ou cationique, en particulier parmi les polyuréthanes polyéthers à séquences hydrophiles et hydrophobes, les polymères à squelette aminoplaste éther comportant au moins une chaîne grasse, les polyuréthanes associatifs cationiques, les dérivés de cellulose quaternisée comportant au moins une chaîne grasse, et les polyvinyllactames cationiques.

Selon un mode de réalisation particulier, le polymère associatif est choisi parmi les alkyl(C₈-C₃₀)hydroxyéthylcelluloses quaternisées et notamment la laurylhydroxyéthylcellulose quaternisée.

La concentration en polymères associatifs dans la composition conforme à l'invention est généralement comprise entre 0,01 à 10 % en poids, de préférence entre 0,1 et 5 % en poids du poids total de la composition.

Le ou les sels peroxygénés utiles à l'invention sont par exemple choisis parmi les persulfates, les perborates, les percarbonates, les peroxydes de métaux alcalins ou alcalino-terreux, et leurs mélanges. De préférence, on utilisera les persulfates et leurs mélanges, par exemple les persulfates de sodium, de potassium et d'ammonium, et leurs mélanges.

La concentration en sels peroxygénés dans la composition conforme à l'invention est généralement comprise entre 1 et 70 % en poids, et de préférence entre 20 et 60 % en poids du poids total de la composition.

Le ou les agents alcalins utiles dans la composition de la présente invention sont par exemple choisis parmi l'urée, les sels d'ammonium comme le chlorure d'ammonium, le sulfate d'ammonium, le phosphate d'ammonium ou le nitrate d'ammonium, les silicates, les phosphates ou les carbonates de métaux alcalins ou alcalino-terreux, tels que le lithium, le sodium, le potassium, le magnésium, le calcium, le baryum, et leurs mélanges. De préférence, le ou les agents alcalins sont choisis parmi le chlorure d'ammonium, les silicates, les carbonates, et leurs mélanges.

La concentration en agents alcalins dans la composition conforme à l'invention est généralement comprise entre 0,01 et 40 % en poids, et de préférence entre 0,1 et 30 % en poids du poids total de la composition.

Selon un mode de réalisation particulier, la composition conforme à l'invention se présente sous forme de pâte. Selon ce mode de réalisation, elle peut de plus comprendre au moins une phase liquide inerte organique.

Par phase liquide, on entend au sens de la présente invention toute phase capable d'écoulement à température ambiante, généralement entre 15 °C et 40 °C, et à pression atmosphérique, sous l'action de son propre poids.

Par liquide inerte organique, on entend au sens de la présente invention un liquide organique chimiquement inerte vis-à-vis du peroxyde d'hydrogène. Dans le cadre de l'invention, un liquide est inerte si la dégradation du peroxyde d'hydrogène en présence de ce liquide est inférieure à 25 % après 15 heures à 100 °C.

A titre d'exemple de phase liquide inerte, on peut citer les polydécènes de formule C₁₀ₙH_{[(20n)+2]} dans laquelle n varie de 3 à 9 et de préférence de 3 à 7, les esters d'alcools gras ou d'acides gras, les esters ou di-esters de sucres d'acides gras en C₁₂-C₂₄, les éthers cycliques ou les esters cycliques, les huiles de silicone, les huiles minérales ou les huiles végétales, ou leurs mélanges.

Les composés de formule C₁₀ₙ H_{[(20n)+2]} avec n variant de 3 à 9 répondent à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe. Ce sont des produits d'hydrogénation des poly-1-décènes.

Parmi ces composés, on préfère selon l'invention ceux pour lesquels dans la formule, n varie de 3 à 7.

On peut citer à titre d'exemple le produit vendu sous la dénomination Silkflo^{®} 366 NF Polydecene par la société Amoco Chemical, ceux vendus sous la dénomination Nexbase® 2002 FG, 2004 FG, 2006 FG et 2008 FG par la société Fortum.

En ce qui concerne les esters d'alcools gras ou d'acides gras, on peut citer à titre d'exemple :
- les esters de monoalcools inférieurs saturés linéaires ou ramifiés en C₃-C₆, avec des acides gras monofonctionnels en C₁₂-C₂₄, ces derniers pouvant être linéaires ou ramifiés, saturés ou insaturés et choisis notamment parmi les oléates, laurates, palmitates, myristates, béhénates, cocoates, stéarates, linoléates, linolénates, caprates, arachidonates, ou leurs mélanges comme notamment les oléo-palmitates, oléo-stéarates, palmito-stéarates. Parmi ces esters, on préfère plus particulièrement utiliser le palmitate d'isopropyle, le myristate d'isopropyle et le stéarate d'octyl dodécyle,
- les esters de monoalcools linéaires ou ramifiés en C₃-C₈, avec des acides gras bifonctionnels en C₈-C₂₄, ces derniers pouvant être linéaires ou ramifiés, saturés ou insaturés, comme par exemple le di-ester isopropylique de l'acide sébacique, appelé aussi sébaçate de diisopropyle,
- les esters de monoalcools linéaires ou ramifiés en C₃-C₈, avec des acides gras bifonctionnels en C₂-C₈, ces derniers pouvant être linéaires ou ramifiés, saturés ou insaturés, comme par exemple l'adipate de di-octyle et le maléate de di-caprylyle,
- l'ester d'un acide trifonctionnnel comme le citrate de tri-éthyle.

En ce qui concerne les esters et di-esters de sucres d'acides gras en C₁₂-C₂₄, on entend par "sucre" des composés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres utilisables selon l'invention, on peut citer par exemple le sucrose (ou saccharose), le glucose, le galactose, le ribose, le fuctose, le maltose, le fructose, le mannose, l'arabinose, le xylose, le lactose, et leurs dérivés notamment alkylés, tels que les dérivés méthylés comme le méthylglucose.

Les esters de sucres et d'acides gras utilisables selon l'invention peuvent être choisis notamment dans le groupe comprenant les esters ou mélanges d'esters de sucres décrits ci-avant et d'acides gras en C₁₂-C₂₄, linéaires ou ramifiés, saturés ou insaturés.

Les esters peuvent être choisis parmi les mono-, di-, tri- et tétra-esters, les polyesters et leurs mélanges.

Ces esters peuvent être par exemple choisis parmi les oléates, laurates, palmitates, myristates, béhénates, cocoates, stéarates, linoléates, linolénates, caprates, arachidonates, ou leurs mélanges comme notamment les esters mixtes oléo-palmitates, oléo-stéarates, palmito-stéarates.

Plus particulièrement, on préfère utiliser les mono- et di- esters et notamment les mono- ou di- oléates, stéarates, béhénates, oléopalmitates, linoléates, linolénates, oléostéarates, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et triester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose ;
- les produits vendus sous la dénomination Ryoto Sugar Esters par exemple référencés B370 et correspondant au béhénate de saccharose formé de 20 % de monoester et 80 % de di-triester-polyester ;
- le mono-di-palmito-stéarate de sucrose commercialisé par la société Goldschmidt sous la dénomination Tegosoft® PSE.

En ce qui concerne les éthers cycliques et esters cycliques, conviennent notamment la γ-butyrolactone, le diméthyl isosorbide, ou le diisopropyl isosorbide.

Les huiles de silicone peuvent aussi être employées comme phase liquide organique inerte.

Plus particulièrement, les huiles de silicone convenables sont des fluides de silicones liquides et non volatiles de viscosité inférieure ou égale à 10 000 mPa.s à 25 °C, la viscosité des silicones étant mesurée selon la norme ASTM 445 Appendice C.

Les huiles de silicone sont définies plus en détail dans l'ouvrage de Walter NOLL "Chemistry and Technology of Silicones" (1968) - Academic Press.

Parmi les huiles de silicone utilisables selon l'invention, on peut citer notamment les huiles de silicones vendues sous les dénominations DC-200 fluid - 5 mPa.s, DC-200 fluid - 20 mPa.s, DC-200 fluid - 350 mPa.s, DC-200 fluid - 1000 mPa.s, DC-200 fluid - 10 000 mPa.s par la société Dow Corning.

Les huiles minérales peuvent aussi être utilisées comme phase liquide inerte organique, comme par exemple l'huile de paraffine.

Les huiles végétales peuvent aussi convenir, et notamment l'huile d'avocat, l'huile d'olive ou la cire liquide de jojoba.

De préférence, la phase liquide inerte organique est choisie dans le groupe formé par les polydécènes de formule C₁₀ₙH_{[(20n)+2]} dans laquelle n varie de 3 à 9 et de préférence de 3 à 7, les esters d'alcools gras ou d'acides gras, et leurs mélanges.

Lorsque la composition conforme à l'invention comprend au moins une phase liquide inerte organique, la teneur en phase liquide inerte organique varie généralement de 5 à 60 % en poids, de préférence de 10 à 50 % en poids par rapport au poids de la pâte anhydre, et encore plus préférentiellement de 15 à 45 %.

Selon un mode de réalisation particulier de l'invention, la composition conforme à l'invention est anhydre.

Dans le cadre de la présente invention, une composition est anhydre lorsqu'elle présente une teneur en eau inférieure à 1 % en poids, et de préférence inférieure à 0,5 % en poids par rapport au poids total de la composition.

Selon un autre mode de réalisation particulier de l'invention, la composition conforme à l'invention est aqueuse. Elle peut alors de plus comprendre du peroxyde d'hydrogène.

Dans ce cas-là, la composition conforme à l'invention est prête à l'emploi et résulte du mélange d'une composition anhydre conforme à l'invention avec une composition aqueuse comprenant ou non du peroxyde d'hydrogène. Son pH est généralement compris entre les valeurs 3 et 11. II est de préférence compris entre 7 et 11.

La composition conforme à la présente invention peut également comprendre divers additifs classiquement utilisés en cosmétique.

La composition conforme à la présente invention peut ainsi comprendre des agents épaississants minéraux ou organiques, des charges telles que des argiles, des liants tels que la vinylpyrrolidone, des lubrifiants comme les stéarates de polyol ou les stéarates de métaux alcalins ou alcalino-terreux, des silices hydrophiles ou hydrophobes, des pigments, des colorants autres que ceux de la présente invention, des agents matifiants comme les oxydes de titane ou encore des agents tensioactifs anioniques, non ioniques, cationiques, amphotères ou zwittérioniques, des agents antioxydants, des agents de pénétration, des agents séquestrants, des tampons, des agents dispersants, des agents filmogènes, des agents conservateurs, des agents opacifiants, des vitamines, des parfums, des polymères anioniques, cationiques, non ioniques, amphotères ou zwittérioniqes, des céramides, des agents de conditionnement tels que par exemple des silicones volatiles ou non volatiles, modifiées ou non modifiées.

Dans le cas où la composition conforme à l'invention comprend du peroxyde d'hydrogène, elle peut également comprendre des agents de contrôle du dégagement d'oxygène tels que le carbonate ou l'oxyde de magnésium.

Les additifs et les agents de contrôle du dégagement d'oxygène tels que définis précédemment peuvent être présents en quantité comprise pour chacun d'eux entre 0,01 et 40 % en poids, de préférence entre 0,1 et 30 % en poids par rapport au poids total de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Le procédé de décoloration et de coloration simultanées conforme à la présente invention consiste à appliquer sur les fibres kératiniques une composition anhydre conforme à l'invention telle que définie précédemment en présence d'une composition aqueuse comprenant ou non du peroxyde d'hydrogène. La composition aqueuse comprenant ou non du peroxyde d'hydrogène peut être ajoutée à la composition anhydre juste au moment de l'emploi. Elle peut aussi être appliquée simultanément ou séquentiellement à la composition anhydre.

La présente invention a également pour objet un dispositif à plusieurs compartiments, caractérisé par le fait qu'il contient au moins deux compositions dont le mélange conduit à une composition aqueuse conforme à l'invention telle que définie précédemment.

Selon un mode de réalisation particulier de l'invention, le dispositif conforme à invention comporte un premier compartiment qui contient une composition (A) comprenant, dans un milieu approprié pour la teinture, au moins un colorant anionique ou non ionique et au moins un polymère associatif tels que définis précédemment, un deuxième compartiment qui contient une composition (B) anhydre comprenant au moins un sel peroxygéné et au moins un agent alcalin tels que définis précédemment, et un troisième compartiment qui contient une composition (C) aqueuse de peroxyde d'hydrogène.

Selon un autre mode de réalisation particulier de l'invention, le dispositif conforme à invention comporte un premier compartiment qui contient une composition (D) comprenant, dans un milieu approprié pour la teinture, au moins un colorant anionique ou non ionique tel que défini précédemment, un deuxième compartiment qui contient une composition (E) anhydre comprenant au moins un polymère associatif, au moins un sel peroxygéné et au moins un agent alcalin tels que définis précédemment, et un troisième compartiment qui contient une composition (C) aqueuse de peroxyde d'hydrogène.

Selon un autre mode de réalisation particulier de l'invention, le dispositif conforme à invention comporte un premier compartiment qui contient une composition (F) anhydre comprenant au moins un colorant anionique ou non ionique, au moins un polymère associatif, au moins un sel peroxygéné et au moins un agent alcalin tels que définis précédemment, et un deuxième compartiment qui contient une composition (C) aqueuse de peroxyde d'hydrogène.

Selon un autre mode de réalisation particulier de l'invention, le dispositif conforme à invention comporte un premier compartiment qui contient une composition (E) anhydre comprenant au moins un polymère associatif, au moins un sel peroxygéné et au moins un agent alcalin tels que définis précédemment, et un deuxième compartiment qui contient une composition (G) comprenant, dans un milieu approprié pour la teinture, au moins un colorant anionique ou non ionique tel que défini précédemment et du peroxyde d'hydrogène.

Selon un autre mode de réalisation particulier de l'invention, le dispositif conforme à invention comporte un premier compartiment qui contient une composition (B) anhydre comprenant au moins un sel peroxygéné et au moins un agent alcalin tels que définis précédemment, et un deuxième compartiment qui contient une composition (H) comprenant, dans un milieu approprié pour la teinture, au moins un colorant anionique ou non ionique et au moins un polymère associatif tels que définis précédemment et du peroxyde d'hydrogène.

Le milieu approprié pour la teinture des compositions (A), (C), (D), (G) et (H) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids par rapport au poids total de la composition tinctoriale, et plus préférentiellement encore entre 5 et 30 % en poids environ.

Les compositions (A) et (D), encore appelée "booster", peuvent être formulées à pH acide, neutre ou alcalin, le pH pouvant varier entre 3 et 12 environ et de préférence entre 4 et 11 environ.

Les compositions (C), (G) et (H) présentent de préférence un pH inférieur à 7, le pH acide garantissant la stabilité du peroxyde d'hydrogène dans cette composition.

Les compositions (A), (C), (D), (G) et (H) peuvent se présenter sous des formes diverses, telles que sous forme de liquides, de crèmes, de gels, ou sous toute autre forme appropriée pour réaliser une teinture des fibres kératiniques.

Les compositions (B), (E) et (F) anhydres peuvent se présenter sous forme de poudre ou de pâte. Dans le cas où elles se présentent sous forme de pâte, elles comprennent de plus une phase liquide inerte organique telle que définie précédemment.

Les compositions (A) à (I) peuvent également renfermer divers additifs classiquement utilisés en cosmétique tels que ceux qui sont décrits précédemment.

Les compositions (C), (G) et (H) peuvent de plus comprendre des agents de contrôle du dégagement d'oxygène tels que définis précédemment.

Le dispositif conforme à la présente invention peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de décolorer et de colorer simultanément les fibres kératiniques à partir d'un procédé conforme à l'invention tel que défini précédemment.

La présente invention a aussi pour objet l'utilisation pour la décoloration et la coloration simultanées des fibres kératiniques d'une composition conforme à l'invention telle que définie précédemment.

Les exemples qui suivent servent à illustrer l'invention sans toutefois présenter un caractère limitatif.

### EXEMPLE

On a préparé les compositions prêtes à l'emploi suivantes :

| **Composition** | **A** | **B** | **C** |
|---|---|---|---|
| 3-méthylamino-4-nitrophénoxyéthanol | 0,5 g | - | - |
| 4-amino-4'-nitroazobenzène ou Disperse Orange 3 | - | 0,5 g | - |
| Sel de sodium de l'acide 5-(2,4-dinitrophénylamino)-2-(phénylamino)benzène sulfonique ou Acid Orange 3 | - | - | 0,5 g |
| Terpolymère acide (méth)acrylique / acrylate d'éthyle / méthacrylate de béhényle oxyéthyléné (25 OE) en émulsion aqueuse (ACULYN 28) | 1,3 g | 1,3 g | 1,3 g |
| Alcool cétylstéarylique oxyéthyléné (33 OE) | 1,4 g | 1,4 g | 1,4 g |
| Alcool cétylstéarylique | 5,7 g | 5,7 g | 5,7 g |
| Acide éthylène diamine tétracétique | 0,06 g | 0,06 g | 0,06 g |
| Persulfate de potassium | 12 g | 12 g | 12 g |
| Disilicate de sodium hydrate | 4,3 g | 4,3 g | 4,3 g |
| Persulfate de sodium | 1,7 g | 1,7 g | 1,7 g |
| Myristate d'isopropyle | 6,2 g | 6,2 g | 6,2 g |
| Cire d'abeille blanchie | 0,3 g | 0,3 g | 0,3 g |
| Huile de vaseline | 0,3 g | 0,3 g | 0,3 g |
| Oxyde de titane (anatase non traité) | 0,3 g | 0,3 g | 0,3 g |
| Copolymère hexaméthyl diisocyanate / polyéthylèneglycol à terminaison alpha-oméga stéaryl polyoxyéthyléné (NUVIS FX1100) | 0,6 g | 0,6 g | 0,6 g |
| Carboxyméthyl amidon (fécule) de pomme de terre, sel de sodium faiblement réticulé | 0,6 g | 0,6 g | 0,6 g |
| Lauryl sulfate de sodium | 1,1 g | 1,1 g | 1,1 g |
| Stéarate de magnésium | 0,6 g | 0,6 g | 0,6 g |
| Oxyde de magnésium | 0,6 g | 0,6 g | 0,6 g |
| Pyrophosphate tétra-sodique, 10 H₂O | 0,02 g | 0,02 g | 0,02 g |
| Acide étidronique, sel tétrasodique en solution aqueuse à 30 % | 0,1 g | 0,1 g | 0,1 g |
| Peroxyde d'hydrogène en solution aqueuse à 50 % (eau oxygénée 200 volumes) | 13,7 g | 13,7 g | 13,7 g |
| Salicylate de sodium | 0,02 g | 0,02 g | 0,02 g |
| Conservateurs | q.s. | q.s. | q.s. |
| Agents de pH | q.s. pH 9,9 | q.s. pH 9,9 | q.s. pH 9,9 |
| Eau désionisée | q.s.p. 100 g | q.s.p. 100 g | q.s.p. 100 g |

Chacune des compositions A, B et C est appliquée sur une mèche de cheveux châtains naturels à une température de 37 °C. Après un temps de pose de 40 minutes, les mèches sont rincées, lavées avec un shampooing standard, rincées à nouveau puis séchées.

Les nuances obtenues sont décrites dans le tableau ci-dessous.

| **Composition** | **A** | **B** | **C** |
|---|---|---|---|
| Nuance | Blond clair doré intense | Blond beige cuivré | Blond beige cuivré |

## Revendications

1. Composition pour la décoloration et la coloration simultanées des fibres kératiniques comprenant :
- au moins un colorant choisi parmi les colorants anioniques et non ioniques, à l'exception du 7-(6'-méthylphénylazo)-1-acétamido-3,6-disulfo-8-hydroxy-naphtalène, des ortho nitro-anilines substituées en méta du groupement amino, de la quinoline, des dérivés quinoliniques, et de leurs sels d'addition ;
- au moins un polymère associatif ;
- au moins un sel peroxygéné ; et
- au moins un agent alcalin.

2. Composition selon la revendication 1, dans laquelle le ou les colorants anioniques sont choisis parmi les colorants directs nitrés acides, les colorants azoïques acides, les colorants aziniques acides, les colorants triarylméthaniques acides, les colorants indoaminiques acides, les colorants naturels acides.

3. Composition selon la revendication 2, dans laquelle le ou les colorants anioniques sont choisis parmi les composés suivants :
| | |
|---|---|
| (C.I. 45380) | Acid Red 87 |
| (C.I. 10316) | Sel de sodium de l'acide 2,4-dinitro-1-naphtol-7-sulfonique |
| (C.I. 10383) | Acid Orange 3 |
| (C.I. 13015) | Acid Yellow 9 / Food Yellow 2 |
| (C.I. 14780) | Direct Red 45 / Food Red 13 |
| (C.I. 13711) | Acid Black 52 |
| (C.I. 13065) | Acid Yellow 36 |
| (C.I. 14700) | Sel de sodium de l'acide 1-hydroxy-2-(2',4'-xylyl-5-sufonatoazo)-naphtalène-4-sulfonique / Food Red 1 |
| (C.I. 14720) | Acid Red 14 / Food Red 3 / Mordant Blue 79 |
| (C. I. 14805) | Acid Brown 4 |
| (C.I. 15510) | Acid Orange 7 / Pigment Orange 17 / Solvent Orange 49 |
| (C.I. 15985) | Food Yellow 3 / Pigment Yellow 104 |
| (C.I. 16185) | Acid Red 27 / Food Red 9 |
| (C.I. 16230) | Acid Orange 10 / Food Orange 4 |
| (C.I. 16250) | Acid Red 44 |
| (C.I. 17200) | Acid Red 33 / Food Red 12 |
| (C.I. 15685) | Acid Red 184 |
| (C.I. 19125) | Acid Violet 3 |
| (C.I. 18055) | Sel de sodium de l'acide 1-hydroxy-2-(4'-acétamido phénylazo)-8-acétamido-naphtalène-3,6-disulfonique / Acid Violet 7 / Food Red 11 |
| (C.I. 18130) | Acid Red 135 |
| (C.I. 19130) | Acid Yellow 27 |
| (C.I. 19140) | Acid Yellow 23 / Food Yellow 4 |
| (C.I. 20170) | 4'-(sulfonato-2",4"-diméthyl)-bis-(2,6-phénylazo)-1,3-dihydroxy benzène / Acid Orange 24 |
| (C.I. 20470) | Sel de sodium de l'acide 1-amino-2-(4'-nitrophénylazo)-7-phénylazo-8-hydroxy-naphtalène-3,6-disulfonique / Acid Black 1 |
| (C.I. 23266) | (4-((4-méthylphényl) sulfonyloxy)-phénylazo)2,2'-diméthyl-4-((2-hydroxy-5,8-disulfonato)naphtylazo)biphényle / Acid Red 111 |
| (C.I. 27755) | Food Black 2 |
| (C.I. 25440) | 1-(4'-sulfonatophénylazo)-4-((2"-hydroxy-3"-acétylamino-6",8"-disulfonato)naphtylazo)-6-sulfonatonaphtalène (sel tétrasodique) / Food Black 1 |
| (C.I. 42080) | Acide 4-β-hydroxyéthylamino-3-nitrobenzènesulfonique |
| (C.I. 42090) | Acid Blue 9 |
| (C.I. 60730) | Acid Violet 43 |
| (C.I. 61570) | Acid Green 25 |
| (C.I. 62045) | Sel de sodium de l'acide 1-amino-4-cyclohexylamino-9,10-anthraquinone 2-sulfonique / Acid Blue 62 |
| (C.I. 62105) | Acid Blue 78 |
| (C.I. 14710) | Sel de sodium de l'acide 4-hydroxy-3((2-méthoxy phényl)-azo)-1-naphtalène sulfonique / Acid Red 4 |
| | Acide 2-pipéridino 5-nitro benzène sulfonique |
| | Acide 2(4'-N,N(2"-hydroxyéthyl)amino-2'-nitro)aniline éthane sulfonique |
| | Acide 4-β-hydroxyéthylamino-3-nitrobenzène sulfonique |
| (C.I. 42640) | Acid Violet 49 |
| (C.I. 42080) | Acid Blue 7 |
| | Acid Blue 156 |
| | Acid Blue 317 |
| (C.I. 58005) | Sel de sodium du 1,2-dihydroxy-3-sulfo-anthraquinone / Mordant Red 3 |
| (C.I. 62055) | Sel de sodium de l'acide 1-amino-9,10-dihydro-9,10-dioxo-4-(phénylamino) 2-anthracène sulfonique / Acid Blue 25 |
| (C.I. 14710) | Sel de sodium de l'acide 4-hydroxy-3-((2-méthoxyphényl)-azo)-1-naphtalène sulfonique / Acid Red 4 |

4. Composition selon l'une quelconque des revendications précédentes, dans laquelle le ou les colorants non ioniques sont choisis parmi les colorants non ioniques benzéniques nitrés, les colorants non ioniques azoïques, azométhiniques, méthiniques, anthraquinoniques, naphtoquinoniques, benzoquinoniques, phénotiaziniques indigoïdes, xanthéniques, phénanthridiniques, phtalocyanines, ceux dérivés du triarylméthane, seuls ou en mélanges.

5. Composition selon la revendication 4, dans laquelle le ou les colorants non ioniques sont choisis parmi le 1-hydroxy-3-nitro-4-N-(γ-hydroxypropyl)amino benzène ; le N-(β-hydroxyéthyl)amino-3-nitro-4-amino benzène ; le 1-hydroxy-3-nitro-4-N-(β-hydroxyéthyl)amino benzène ; le 1,4-diamino-2-nitrobenzène ; le 1-amino-2-nitro-4-méthylamino benzène ; la N-(β-hydroxyéthyl)-2-nitro-paraphénylènediamine ; la 2-nitro-4-amino-diphénylamine ; le 1-amino-3-nitro-6-hydroxybenzène ; le 1-(β-aminoéthyl)amino-2-nitro-4-(β-hydroxyéthyloxy) benzène ; le 1-(β, γ-dihydroxypropyl)oxy-3-nitro-4-(β-hydroxyéthyl)amino benzène ; le 1-hydroxy-3-nitro-4-aminobenzène ; le 1-hydroxy-2-amino-4,6-dinitrobenzène ; le 1-méthoxy-3-nitro-4-(β-hydroxyéthyl)amino benzène ; la 2-nitro-4'-hydroxydiphénylamine ; le 1-(β-hydroxyéthyl)amino-2-méthoxy-4-nitrobenzène ; le 1-amino-2-nitro-6-méthyl-benzène ; le 1-(β-hydroxyéthyl)amino-2-hydroxy-4-nitrobenzène ; la N-(β-hydroxyéthyl)-2-nitro-4-trifluorométhylaniline ; le 4-(β-hydroxyéthyl)amino-3-nitro-chlorobenzène ; le 4-(β-hydroxyéthyl)amino-3-nitro-méthylbenzène ; le 4-(β,γ -dihydroxypropyl)amino-3-nitro-trifluorométhylbenzène ; le 1-(β-uréidoéthyl)amino-4-nitrobenzène ; le 1-hydroxy-2-amino-5-nitrobenzène ; le 1-amino-2-[tris(hydroxyméthyl)méthyl]amino-5-nitro-benzène ; le 1-(β-hydroxyéthyl)amino-2-nitrobenzène ; le 4-(β-hydroxyéthyl)amino-3-nitrobenzamide ; le 1-(β-hydroxyéthyl)amino-4-N,N-bis-(β-hydroxyéthyl)amino 2-nitrobenzène ; le 1-(γ-hydroxypropyl)amino 4-N,N-bis-(β-hydroxyéthyl)amino 2-nitrobenzène ; le 1-(β-hydroxyéthyl)amino 4-(N-méthyl, N-β-hydroxyéthyl)amino 2-nitrobenzène ; le 1-(β-hydroxyéthyl)amino 4-(N-éthyl, N-β-hydroxyéthyl)amino 2-nitrobenzène ; le 1-(β,γ-dihydroxypropyl)amino 4-(N-éthyl, N-β-hydroxyéthyl)amino 2-nitrobenzène ; le Disperse Orange 3 ; le Disperse Red 17 ; le Disperse Violet 4 ; le Disperse Violet 8 ; le Disperse Blue 1 ; le Disperse Red 15 ; le Solvent Violet 13 ; le Solvent Violet 11 ; le Disperse Blue 3 ; le Disperse Blue 7 ; le Disperse Red 11 ; le Natural Brown 7 ; le Disperse Black 9 ; le Disperse Violet 15 ; le Natural Orange 6 ; la 2-hydroxy 3-méthoxy 1,4-naphtoquinone ; la 3-hydroxy 2-méthyl 1,4-naphtoquinone ou le phtiocol ; la 3,6-dihydroxy 5-méthoxy p-toluquinone ou la spinulosine ; le HC Blue 14.

6. Composition selon l'une quelconque des revendications précédentes, dans laquelle la concentration en colorants anioniques et / ou non ioniques est comprise entre 0,0001 et 10 % en poids du poids total de la composition.

7. Composition selon l'une quelconque des revendications précédentes, dans laquelle le ou les polymères associatifs sont choisis dans le groupe formé par ceux de type non ionique ou cationique.

8. Composition selon la revendication 7, dans laquelle le ou les polymères associatifs de type non ionique sont choisis dans le groupe formé par les polyuréthanes polyéthers à séquences hydrophiles et hydrophobes, et les polymères à squelette aminoplaste éther comportant au moins une chaîne grasse.

9. Composition selon la revendication 7, dans laquelle le ou les polymères associatifs de type cationique sont choisis dans le groupe formé par les polyuréthanes associatifs cationiques, les dérivés de cellulose quatemisée comportant au moins une chaîne grasse, et les polyvinyllactames cationiques.

10. Composition selon la revendication 9, dans laquelle les dérivés de cellulose quatemisée comportant au moins une chaîne grasse sont choisis parmi les alkyl (C₈-C₃₀)hydroxyéthylcelluloses quatemisées.

11. Composition selon la revendication 10, dans laquelle le dérivé de cellulose quaternisée comportant au moins une chaîne grasse est la laurylhydroxyéthylcellulose quaternisée.

12. Composition selon l'une quelconque des revendications précéddentes dans laquelle la concentration en polymères associatifs est comprise entre 0,01 à 10% en poids du poids total de la composition.

13. Composition selon l'une quelconque des revendications précédentes, dans laquelle le ou les sels peroxygénés sont choisis parmi les persulfates, les perborates, les percarbonates, les peroxydes de métaux alcalins ou alcalino-terreux, et leurs mélanges.

14. Composition selon la revendication 13, dans laquelle le ou les sels peroxygénés sont choisis parmi les persulfates et leurs mélanges.

15. Composition selon la revendication 14, dans laquelle le ou les sels peroxygénés sont choisis parmi le persulfate de sodium, le persulfate de potassium, le persulfate d'ammonium, et leurs mélanges.

16. Composition selon l'une quelconque des revendications précédentes, dans laquelle la concentration en sels peroxygénés est comprise entre 1 et 70 % en poids du poids total de la composition.

17. Composition selon l'une quelconque des revendications précédentes, dans laquelle le ou les agents alcalins sont choisis parmi l'urée, le chlorure d'ammonium, le sulfate d'ammonium, le phosphate d'ammonium, le nitrate d'ammonium, les silicates, les phosphates ou les carbonates de métaux alcalins ou alcalino-terreux, et leurs mélanges.

18. Composition selon l'une quelconque des revendications précédentes, dans laquelle la concentration en agents alcalins est comprise entre 0,01 et 40 % en poids du poids total de la composition.

19. Composition selon l'une quelconque des revendications précédentes, comprenant de plus au moins une phase liquide inerte organique.

20. Composition selon la revendication 19, dans laquelle la ou les phases liquides inertes organiques sont choisies parmi par les polydécènes de formule C₁₀ₙH_{[(20n)+2]} dans laquelle n varie de 3 à 9, les esters d'alcools gras ou d'acides gras, les esters ou di-esters de sucres d'acides gras en C₁₂-C₂₄, les éthers cycliques ou les esters cycliques, les huiles de silicone, les huiles minérales, les huiles végétales, et leurs mélanges.

21. Composition selon la revendication 20, dans laquelle la ou les phases liquides inertes organiques sont choisies parmi les polydécènes de formule C₁₀ₙH_{[(20n)+2]} dans laquelle n varie de 3 à 9, les esters d'alcools gras ou d'acides gras, et leurs mélanges.

22. Composition selon l'une quelconque des revendications 19 à 21, dans laquelle la concentration en phases liquides inertes organiques est comprise entre 5 et 60 % en poids du poids total de la composition.

23. Composition selon l'une quelconque des revendications précédentes, **caractérisée en ce qu'**elle est anhydre.

24. Composition selon l'une quelconque des revendications 1 à 22, **caractérisée en ce qu'**elle est aqueuse.

25. Composition selon la revendication 24, comprenant de plus du peroxyde d'hydrogène.

26. Procédé de décoloration et de coloration simultanées des fibres kératiniques, **caractérisé par le fait que** l'on applique sur lesdites fibres kératiniques une composition anhydre telle que définie à la revendication 23 en présence d'une composition aqueuse comprenant ou non du peroxyde d'hydrogène.

27. Dispositif à plusieurs compartiments, **caractérisé par le fait qu'**il contient au moins deux compositions dont le mélange conduit à une composition aqueuse telle que définie à la revendication 24 ou 25.

28. Utilisation pour la décoloration et la coloration simultanées d'une composition telle que définie à l'une quelconque des revendications 1 à 25.

## Claims

1. Composition for simultaneously bleaching and dyeing keratin fibres, comprising:
- at least one dye chosen from anionic and nonionic dyes, with the exception of 7-(6'-methylphenylazo)-1-acetamido-3,6-disulfo-8-hydroxynaphthalene, ortho-nitroanilines substituted meta to the amino group, quinoline and quinoline derivatives, and addition salts thereof;
- at least one associative polymer;
- at least one peroxygenated salt; and
- at least one alkaline agent.

2. Composition according to Claim 1, in which the anionic dye(s) is (are) chosen from acidic nitro direct dyes, acidic azo dyes, acidic azine dyes, acidic triarylmethane dyes, acidic indoamine dyes and acidic natural dyes.

3. Composition according to Claim 2, in which the anionic dye(s) is (are) chosen from the following compounds:
| | |
|---|---|
| (C.I. 45380) | Acid Red 87 |
| (C.I. 10316) | Sodium salt of 2,4-dinitro-1-naphthol-7-sulfonic acid |
| (C.I. 10383) | Acid Orange 3 |
| (C.I. 13015) | Acid Yellow 9/Food Yellow 2 |
| (C.I. 14780) | Direct Red 45/Food Red 13 |
| (C.I. 13711) | Acid Black 52 |
| (C.I. 13065) | Acid Yellow 36 |
| (C.I. 14700) | Sodium salt of 1-hydroxy-2-(2',4'-xylyl-5-sulfonatoazo)naphthalene-4-sulfonic acid/Food Red 1 |
| (C.I. 14720) | Acid Red 14/Food Red 3/Mordant Blue 79 |
| (C.I. 14805) | Acid Brown 4 |
| (C.I. 15510) | Acid Orange 7/Pigment Orange 17/Solvent Orange 49 |
| (C.I. 15985) | Food Yellow 3/Pigment Yellow 104 |
| (C.I. 16185) | Acid Red 27/Food Red 9 |
| (C.I. 16230) | Acid Orange 10/Food Orange 4 |
| (C.I. 16250) | Acid Red 44 |
| (C.I. 17200) | Acid Red 33/Food Red 12 |
| (C.I. 15685) | Acid Red 184 |
| (C.I. 19125) | Acid Violet 3 |
| (C.I. 18055) | Sodium salt of 1-hydroxy-2-(4'-acetamido-phenylazo)-8-acetamidonaphthalene-3,6-disulfonic acid/Acid Violet 7/Food Red 11 |
| (C.I. 18130) | Acid Red 135 |
| (C.I. 19130) | Acid Yellow 27 |
| (C.I. 19140) | Acid Yellow 23/Food Yellow 4 |
| (C.I. 20170) | 4'-(Sulfonato-2",4"-dimethyl)bis(2,6-phenyl-azo)-1,3-dihydroxybenzene/Acid Orange 24 |
| (C.I. 20470) | Sodium salt of 1-amino-2-(4'-nitrophenyl-azo)-7-phenylazo-8-hydroxynaphthalene-3,6-disulfonic acid/Acid Black 1 |
| (C.I. 23266) | (4-((4-Methylphenyl)sulfonyloxy)phenylazo)-2,2'-dimethyl-4-((2-hydroxy-5,8-disulfon-ato)naphthylazo)biphenyl/Acid Red 111 |
| (C.I. 27755) | Food Black 2 |
| (C.I. 25440) | 1-(4'-Sulfonatophenylazo)-4-((2"-hydroxy-3"-acetylamino-6",8"-disulfonato)naphthyl-azo)-6-sulfonatonaphthalene (tetrasodium salt)/Food Black 1 |
| (C.I. 42080) | 4-β-Hydroxyethylamino-3-nitrobenzenesulfon-ic acid |
| (C.I. 42090) | Acid Blue 9 |
| (C.I. 60730) | Acid Violet 43 |
| (C.I. 61570) | Acid Green 25 |
| (C.I. 62045) | Sodium salt of 1-amino-4-cyclohexylamino-9, 10-anthraquinone-2-sulfonic acid/Acid Blue 62 |
| (C.I. 62105) | Acid Blue 78 |
| (C.I. 14710) | Sodium salt of 4-hydroxy-3-((2-methoxyphenyl)azo)-1-naphthalenesulfonic acid/Acid Red 4 |
| | 2-Piperidino-5-nitrobenzenesulfonic acid |
| | 2-(4'-N,N-(2"-Hydroxyethyl)amino-2'-nitro)-anilinethanesulfonic acid |
| | 4-β-Hydroxyethylamino-3-nitrobenzenesulfonic acid |
| (C.I. 42640) | Acid Violet 49 |
| (C.I. 42080) | Acid Blue 7 |
| | Acid Blue 156 |
| | Acid Blue 317 |
| (C.I. 58005) | Sodium salt of 1,2-dihydroxy-3-sulfoanthraquinone/Mordant Red 3 |
| (C.I. 62055) | Sodium salt of 1-amino-9,10-dihydro-9,10-dioxo-4-(phenylamino)-2-anthracenesulfonic acid/Acid Blue 25 |
| (C.I. 14710) | Sodium salt of 4-hydroxy-3-((2-methoxyphenyl)azo)-1-naphthalenesulfonic acid/Acid Red 4 |

4. Composition according to any one of the preceding claims, in which the nonionic dye(s) is (are) chosen from nonionic nitrobenzene dyes and nonionic azo, azomethine, methine, anthraquinone, naphthoquinone, benzoquinone, phenothiazine, indigoid, xanthene, phenanthridine, phthalocyanin and triarylmethane-based dyes, alone or as mixtures.

5. Composition according to Claim 4, in which the nonionic dye(s) is (are) chosen from 1-hydroxy-3-nitro-4-N-(γ-hydroxypropyl)aminobenzene; N-(β-hydroxyethyl)-amino-3-nitro-4-aminobenzene; 1-hydroxy-3-nitro-4-N-(β-hydroxyethyl)aminobenzene; 1,4-diamino-2-nitrobenzene; 1-amino-2-nitro-4-methylaminobenzene; N-(β-hydroxyethyl)-2-nitro-para-phenylenediamine; 2-nitro-4-amino-diphenylamine; 1-amino-3-nitro-6-hydroxybenzene; 1-(β-aminoethyl)amino-2-nitro-4-(β-hydroxyethyloxy)benzene; 1-(β,γ-dihydroxypropyl)oxy-3-nitro-4-(β-hydroxyethyl)-aminobenzene; 1-hydroxy-3-nitro-4-aminobenzene; 1-hydroxy-2-amino-4,6-dinitrobenzene; 1-methoxy-3-nitro-4-(β-hydroxyethyl)aminobenzene; 2-nitro-4'-hydroxydiphenylamine; 1-(β-hydroxyethyl)amino-2-methoxy-4-nitrobenzene; 1-amino-2-nitro-6-methylbenzene; 1-(β-hydroxyethyl)amino-2-hydroxy-4-nitrobenzene; N-(β-hydroxyethyl)-2-nitro-4-trifluoromethylaniline; 4-(β-hydroxyethyl)amino-3-nitrochlorobenzene; 4-(β-hydroxyethyl)amino-3-nitromethylbenzene; 4-(β,γ-dihydroxypropyl)amino-3-nitrotrifluoromethylbenzene; 1-(β-ureidoethyl)amino-4-nitrobenzene; 1-hydroxy-2-amino-5-nitrobenzene; 1-amino-2-[tris(hydroxymethyl)methyl]amino-5-nitrobenzene; 1-(β-hydroxyethyl)amino-2-nitrobenzene; 4-(β-hydroxyethyl)amino-3-nitrobenzamide; 1-(β-hydroxyethyl)amino-4-N,N-bis(β-hydroxyethyl)amino-2-nitrobenzene; 1-(γ-hydroxypropyl)amino-4-N,N-bis(β-hydroxyethyl)amino-2-nitrobenzene; 1-(β-hydroxyethyl)amino-4-(N-methyl-N-β-hydroxyethyl)amino-2-nitrobenzene; 1-(β-hydroxyethyl)amino-4-(N-ethyl-N-β-hydroxyethyl)amino-2-nitrobenzene; 1-(β,γ-dihydroxypropyl)amino-4-(N-ethyl-N-β-hydroxyethyl)amino-2-nitrobenzene; Disperse Orange 3; Disperse Red 17; Disperse Violet 4; Disperse Violet 8; Disperse Blue 1; Disperse Red 15; Solvent Violet 13; Solvent Violet 11; Disperse Blue 3; Disperse Blue 7; Disperse Red 11; Natural Brown 7; Disperse Black 9; Disperse Violet 15; Natural Orange 6; 2-hydroxy-3-methoxy-1,4-naphthoquinone; 3-hydroxy-2-methyl-1,4-naphthoquinone or phthiocol; 3,6-dihydroxy-5-methoxy-p-toluquinone or spinulosin; HC Blue 14.

6. Composition according to any one of the preceding claims, in which the concentration of anionic and/or nonionic dyes is between 0.0001% and 10% by weight relative to the total weight of the composition.

7. Composition according to any one of the preceding claims, in which the associative polymer(s) is (are) chosen from the group formed by those of nonionic or cationic type.

8. Composition according to Claim 7, in which the associative polymer(s) of nonionic type is (are) chosen from the group formed by polyether polyurethanes containing hydrophilic and hydrophobic blocks, and polymers with an aminoplast ether backbone comprising at least one fatty chain.

9. Composition according to Claim 7, in which the associative polymer(s) of cationic type is (are) chosen from the group formed by cationic associative polyurethanes, quaternized cellulose derivatives comprising at least one fatty chain and cationic polyvinyllactams.

10. Composition according to Claim 9, in which the quaternized cellulose derivatives comprising at least one fatty chain are chosen from quaternized (C₈-C₃₀)alkylhydroxyethylcelluloses.

11. Composition according to Claim 10, in which the quaternized cellulose derivative comprising at least one fatty chain is quaternized laurylhydroxyethylcellulose.

12. Composition according to any one of the preceding claims, in which the concentration of associative polymers is between 0.01% and 10% by weight relative to the total weight of the composition.

13. Composition according to any one of the preceding claims, in which the peroxygenated salt(s) is (are) chosen from alkali metal or alkaline-earth metal persulfates, perborates, percarbonates and peroxides, and mixtures thereof.

14. Composition according to Claim 13, in which the peroxygenated salt(s) is (are) chosen from persulfates and mixtures thereof.

15. Composition according to Claim 14, in which the peroxygenated salt(s) is (are) chosen from sodium persulfate, potassium persulfate and ammonium persulfate, and mixtures thereof.

16. Composition according to any one of the preceding claims, in which the concentration of peroxygenated salts is between 1% and 70% by weight relative to the total weight of the composition.

17. Composition according to any one of the preceding claims, in which the alkaline agent(s) is (are) chosen from urea, ammonium chloride, ammonium sulfate, ammonium phosphate, ammonium nitrate, and alkali metal or alkaline-earth metal silicates, phosphates or carbonates, and mixtures thereof.

18. Composition according to any one of the preceding claims, in which the concentration of alkaline agents is between 0.01% and 40% by weight relative to the total weight of the composition.

19. Composition according to any one of the preceding claims, also comprising at least one inert organic liquid phase.

20. Composition according to Claim 19, in which the inert organic liquid phase(s) is (are) chosen from the polydecenes of formula C₁₀ₙH_{[(20n)+2]} in which n ranges from 3 to 9, esters of fatty alcohols or of fatty acids, sugar esters or diesters of C₁₂-C₂₄ fatty acids, cyclic ethers or cyclic esters, silicone oils, mineral oils and plant oils, and mixtures thereof.

21. Composition according to Claim 20, in which the inert organic liquid phase(s) is (are) chosen from the polydecenes of formula C₁₀ₙH_{[(20n)+2]} in which n ranges from 3 to 9, and esters of fatty alcohols or of fatty acids, and mixtures thereof.

22. Composition according to any one of Claims 19 to 21, in which the concentration of inert organic liquid phases is between 5% and 60% by weight relative to the total weight of the composition.

23. Composition according to any one of the preceding claims, **characterized in that** it is anhydrous.

24. Composition according to any one of Claims 1 to 22, **characterized in that** it is aqueous.

25. Composition according to Claim 24, also comprising hydrogen peroxide.

26. Process for simultaneously bleaching and dyeing keratin fibres, **characterized in that** an anhydrous composition as defined in Claim 23 is applied to the said keratin fibres in the presence of an aqueous composition optionally comprising hydrogen peroxide.

27. Multi-compartment device, **characterized in that** it contains at least two compositions, the mixing of which leads to an aqueous composition as defined in Claim 24 or 25.

28. Use, for simultaneously bleaching and dyeing, of a composition as defined in any one of Claims 1 to 25.

## Patentansprüche

1. Zusammensetzung zum Entfärben und gleichzeitigen Färben von Keratinfasern, die enthält:
- mindestens einen Farbstoff, der unter den anionischen und nichtionischen Farbstoffen mit Ausnahme von 7-(6'-Methylphenylazo)-1-acetamido-3,6-disulfo-8-hydroxynaphthalin, in meta-Stellung der Aminogruppe substituierten ortho-Nitroanilinen, Chinolin, Chinolinderivaten und deren Additionssalzen ausgewählt ist;
- mindestens ein assoziatives Polymer;
- mindestens ein Peroxysalz; und
- mindestens eine Base.

2. Zusammensetzung nach Anspruch 1, bei der der oder die anionische(n) Farbstoff(e) unter den sauren nitrierten Direktfarbstoffen, sauren Azofarbstoffen, sauren Azinfarbstoffen, sauren Triarylmethanfarbstoffen, sauren Indoaminfarbstoffen und sauren natürlichen Farbstoffen ausgewählt sind.

3. Zusammensetzung nach Anspruch 2, bei der der oder die anionische(n) Farbstoff(e) unter den folgenden Verbindungen ausgewählt sind:
| | |
|---|---|
| (C.I. 45380) | Acid Red 87 |
| (C.I. 10316) | Natriumsalz der 2,4-Dinitro-1-naphthol-7-sulfonsäure |
| (C.I. 10383) | Acid Orange 3 |
| (C.I. 13015) | Acid Yellow 9 / Food Yellow 2 |
| (C.I. 14780) | Direct Red 45 / Food Red 13 |
| (C.I. 13711) | Acid Black 52 |
| (C.I. 13065) | Acid Yellow 36 |
| (C.I. 14700) | Natriumsalz der 1-Hydroxy-2-(2',4'-xylyl-5-sufonatoazo)-naphthalin-4-sulfonsäure/Food Red 1 |
| (C.I. 14720) | Acid Red 14/Food Red 3/Mordant Blue 79 |
| (C. I. 14805) | Acid Brown 4 |
| (C.I. 15510) | Acid Orange 7/Pigment Orange 17/Solvent Orange 49 |
| (C.I. 15985) | Food Yellow 3 / Pigment Yellow 104 |
| (C.I. 16185) | Acid Red 27 / Food Red 9 |
| (C.I. 16230) | Acid Orange 10 / Food Orange 4 |
| (C.I. 16250) | Acid Red 44 |
| (C.I. 17200) | Acid Red 33 / Food Red 12 |
| (C.I. 15685) | Acid Red 184 |
| (C.I. 19125) | Acid Violet 3 |
| (C.I. 18055) | Natriumsalz der 1-Hydroxy-2-(4'-acetamido phenylazo)-8-acetamido-naphthalin-3,6-disulfonsäure/Acid Violet 7/Food Red 11 |
| (C.I. 18130) | Acid Red 135 |
| (C.I. 19130) | Acid Yellow 27 |
| (C.I. 19140) | Acid Yellow 23/Food Yellow 4 |
| (C.I. 20170) | 4'-(Sulfonato-2",4"-dimethyl)-bis-(2,6-phenylazo)-1,3-dihydroxy-benzol/Acid Orange 24 |
| (C.I. 20470) | Natriumsalz der 1-Amino-2-(4'-nitrophenyl-azo)-7-phenylazo-8-hydroxy-naphthalin-3,6-disulfonsäure/Acid Black 1 |
| (C.I. 23266) | (4-((4-Methylphenyl)sulfonyloxy)-phenylazo)-2,2'-dimethyl-4-((2-hydroxy-5,8-disulfonato)-naphtylazo)biphenyl/Acid Red 111 |
| (C.I. 27755) | Food Black 2 |
| (C.I. 25440) | 1-(4'-Sulfonatophenylazo)-4-((2"-hydroxy-3"-acetylamino-6",8"-disulfonato)naphtylazo)-6-sulfonatonaphthalin (Tetranatriumsalz)/Food Black 1 |
| (C.I. 42080) | 4-β-Hydroxyethylamino-3-nitrobenzolsulfonsäure |
| (C.I. 42090) | Acid Blue 9 |
| (C.I. 60730) | Acid Violet 43 |
| (C.I. 61570) | Acid Green 25 |
| (C.I. 62045) | Natriumsalz der 1-Amino-4-cyclohexylamino-9,10-anthrachinon-2-sulfonsäure/Acid Blue 62 |
| (C.I. 62105) | Acid Blue 78 |
| (C.I. 14710) | Natriumsalz der 4-Hydroxy-3-((2-methoxyphenyl)-azo)-1-naphthalin-sulfonsäure/Acid Red 4 |
| | 2-Piperidino-5-nitro-benzolsulfonsäure |
| | 2-(4'-N,N-(2"-Hydroxyethyl)amino-2'-nitro)anilin-ethansulfonsäure |
| | 4-β-Hydroxyethylamino-3-nitrobenzolsulfonsäure |
| (C.I. 42640) | Acid Violet 49 |
| (C.I. 42080) | Acid Blue 7 |
| | Acid Blue 156 |
| | Acid Blue 317 |
| (C.I. 58005) | Natriumsalz von 1,2-Dihydroxy-3-sulfoanthrachionon/Mordant Red 3 |
| (C.I. 62055) | Natriumsalz der 1-Amino-9,10-dihydro-9,10-dioxo-4-(phenylamino)-2-anthracensulfonsäure/Acid Blue 25 |
| (C.I. 14710) | Natriumsalz der 4-Hydroxy-3-((2-methoxyphenyl)-azo)-1-naphthalin-sulfonsäure/Acid Red 4 |

4. Zusammensetzung nach einem der vorhergehenden Ansprüche, bei der der oder die nichtionische(n) Farbstoff(e) unter den nichtionischen nitrierten Benzolfarbstoffen, nichtionischen Azofarbstoffen, Azomethin-Farbstoffen, Methin-Farbstoffen, Anthrachinon-Farbstoffen, Naphthochinon-Farbstoffen, Benzochinon-Farbstoffen, Phenothiazinindigoiden, Xanthen-Farbstoffen, Phenanthridin-Farbstoffen, Phthalocyaninen, von Triarylmethan abgeleiteten Farbstoffen oder deren Gemischen ausgewählt sind.

5. Zusammensetzung nach Anspruch 4, bei der der oder die nichtionische(n) Farbstoff(e) unter den folgenden Verbindungen ausgewählt sind: 1-Hydroxy-3-nitro-4-N-(y-hydroxypropyl)aminobenzol; N-(β-Hydroxyethyl)amino-3-nitro-4-amino-benzol; 1-Hydroxy-3-nitro-4-N-(β-hydroxyethyl)amino-benzol; 1,4-Diamino-2-nitro-benzol; 1-Amino-2-nitro-4-methylamino-benzol; N-(β-Hydroxyethyl)-2-nitro-paraphenylendiamin; 2-Nitro-4-amino-diphenylamin; 1-Amino-3-nitro-6-hydroxy-benzol; 1-(β-Aminoethyl)amino-2-nitro-4-(β-hydroxyethyloxy)-benzol; 1-(β,γ-Dihydroxypropyl)oxy-3-nitro-4-(β-hydroxyethyl)amino-benzol; 1-Hydroxy-3-nitro-4-amino-benzol; 1-Hydroxy-2-amino-4,6-dinitro-benzol; 1-Methoxy-3-nitro-4-(β-hydroxyethyl)amino-benzol; 2-Nitro-4'-hydroxydiphenylamin; 1-(β-Hydroxyethyl)amino-2-methoxy-4-nitro-benzol; 1-Amino-2-nitro-6-methyl-benzol; 1-(β-Hydroxyethyl)amino-2-hydroxy-4-nitro-benzol; N-(β-Hydroxyethyl)-2-nitro-4-trifluormethylanilin; 4-(β-Hydroxyethyl)amino-3-nitro-chlor-benzol; 4-(β-Hydroxyethyl)amino-3-nitro-methylbenzol; 4-(β,γ-Dihydroxypropyl)amino-3-nitro-trifluormethyl-benzol; 1-(β-Ureidoethyl)amino-4-nitro-benzol; 1-Hydroxy-2-amino-5-nitro-benzol; 1-Amino-2-[tris(hydroxymethyl)methyl]-amino-5-nitro-benzol; 1-(β-Hydroxyethyl)amino-2-nitrobenzol; 4-(β-Hydroxyethyl)amino-3-nitrobenzamid; 1-(β-Hydroxyethyl)amino-4-N,N-bis(β-hydroxyethyl)amino-2-nitro-benzol; 1-(γ-Hydroxypropyl)amino-4-N,N-bis(β-hydroxyethyl)amino-2-nitrobenzol; 1-(β-Hydroxyethyl)amino-4-(N-methyl, N-β-Hydroxyethyl)-amino-2-nitro-benzol; 1-(β-Hydroxyethyl)amino-4-(N-ethyl, N-β-hydroxyethyl)amino-2-nitro-benzol; 1-(β,γ-Dihydroxypropyl)amino 4-(N-ethyl, N-β-hydroxyethyl)amino-2-nitro-benzol; Disperse Orange 3; Disperse Red 17; Disperse Violet 4; Disperse Violet 8; Disperse Blue 1; Disperse Red 15; Solvent Violet 13; Solvent Violet 11; Disperse Blue 3; Disperse Blue 7; Disperse Red 11; Natural Brown 7; Disperse Black 9; Disperse Violet 15; Natural Orange 6; 2-Hydroxy 3-methoxy-1,4-naphthochinon; 3-Hydroxy-2-methyl-1,4-naphthochinon oder Phtiocol; 3,6-Dihydroxy-5-methoxy-p-toluchinon oder Spinulosin; HC Blue 14.

6. Zusammensetzung nach einem der vorhergehenden Ansprüche, wobei die Konzentration der anionischen und/oder nichtionischen Farbstoffe im Bereich von 0,0001 bis 10 Gew.-% des Gesamtgewichts der Zusammensetzung liegt.

7. Zusammensetzung nach einem der vorhergehenden Ansprüche, bei der das oder die assoziative(n) Polymer(e) unter den Polymeren vom nichtionischen oder kationischen Typ ausgewählt sind.

8. Zusammensetzung nach Anspruch 7, bei der das oder die assoziative(n) Polymer(e) vom nichtionischen Typ unter den Polyurethanpolyethern mit hydrophilen und hydrophoben Sequenzen und den Polymeren mit Aminoplastether-Gerüst, die mindestens eine Fettkette aufweisen, ausgewählt sind.

9. Zusammensetzung nach Anspruch 7, bei der das oder die assoziative(n) Polymer(e) vom kationischen Typ unter den kationischen assoziativen Polyurethanen, quaternisierten Cellulosederivaten, die mindestens eine Fettkette aufweisen, und kationischen Polyvinyllactamen ausgewählt sind.

10. Zusammensetzung nach Anspruch 9, wobei die quaternisierten Cellulosederivate, die mindestens eine Fettkette aufweisen, unter den quaternisierten Alkyl(C₈-₃₀)hydroxyethylcellulosen ausgewählt sind.

11. Zusammensetzung nach Anspruch 10, wobei das quaternisierte Cellulosederivat, das mindestens eine Fettkette aufweist, die Laurylhydroxyethylcellulose ist.

12. Zusammensetzung nach einem der vorhergehenden Ansprüche, wobei die Konzentration der assoziativen Polymere im Bereich von 0,01 bis 10 Gew.-% des Gesamtgewichts der Zusammensetzung liegt.

13. Zusammensetzung nach einem der vorhergehenden Ansprüche, bei der das oder die Peroxysalz(e) unter den Persulfaten, Perboraten, Percarbonaten und Peroxiden von Alkalimetallen und Erdalkalimetallen und deren Gemischen ausgewählt sind.

14. Zusammensetzung nach Anspruch 13, wobei das oder die Peroxysalz(e) unter den Persulfaten und deren Gemischen ausgewählt sind.

15. Zusammensetzung nach Anspruch 14, wobei das oder die Peroxysalz(e) unter Natriumpersulfat, Kaliumpersulfat, Ammoniumpersulfat und deren Gemischen ausgewählt sind.

16. Zusammensetzung nach einem der vorhergehenden Ansprüche, bei der die Konzentration der Peroxysalze im Bereich von 1 bis 70 Gew.-% des Gesamtgewichts der Zusammensetzung liegt.

17. Zusammensetzung nach einem der vorhergehenden Ansprüche, bei der die Base(n) unter Harnstoff, Ammoniumchlorid, Ammoniumsulfat, Ammoniumphosphat, Ammoniumnitrat, Silicaten, Phosphaten oder Carbonaten von Alkali- oder Erdalkalimetallen und deren Gemischen ausgewählt sind.

18. Zusammensetzung nach einem der vorhergehenden Ansprüche, wobei die Konzentration der Basen im Bereich von 0,01 bis 40 Gew.-% des Gesamtgewichts der Zusammensetzung liegt.

19. Zusammensetzung nach einem der vorhergehenden Ansprüche, die ferner mindestens eine inerte flüssige organische Phase enthält.

20. Zusammensetzung nach Anspruch 19, bei der die inerte(n), flüssige(n) organische(n) Phase(n) unter den Polydecenen der Formel C₁₀ₙH_{[(20n)+2]}, worin n im Bereich von 3 bis 9 liegt, den Estern von Fettalkoholen oder Fettsäuren, Estern oder Diestern von Zuckern und Fettsäuren mit 12 bis 24 Kohlenstoffatomen, cyclischen Ethern oder cyclischen Estern, Siliconölen, Mineralölen, pflanzlichen Ölen und deren Gemischen ausgewählt sind.

21. Zusammensetzung nach Anspruch 20, bei der die inerte(n), flüssige(n) organische(n) Phase(n) unter den Polydecenen der Formel C₁₀ₙH_{[(20n)+2]}, worin n im Bereich von 3 bis 9 liegt, den Estern von Fettalkoholen oder Fettsäuren und deren Gemischen ausgewählt sind.

22. Zusammensetzung nach einem der Ansprüche 19 bis 21, wobei die Konzentration der inerten, flüssigen organischen Phasen im Bereich von 5 bis 60 Gew.-% des Gesamtgewichts der Zusammensetzung liegt.

23. Zusammensetzung nach einem der vorhergehenden Ansprüche, **dadurch gekennzeichnet, dass** sie wasserfrei ist.

24. Zusammensetzung nach einem der 1 bis 22 Ansprüche, **dadurch gekennzeichnet, dass** sie wässrig ist.

25. Zusammensetzung nach Anspruch 24, die ferner Wasserstoffperoxid enthält.

26. Verfahren zum Entfärben und gleichzeitigen Färben von Keratinfasern, **dadurch gekennzeichnet, dass** eine wasserfreie Zusammensetzung, wie sie in Anspruch 23 definiert ist, auf die Keratinfasern in Gegenwart einer wässrigen Zusammensetzung aufgebracht wird, die gegebenenfalls Wasserstoffperoxid enthält.

27. Vorrichtung mit mehreren Abteilungen, **dadurch gekennzeichnet, dass** sie mindestens zwei Zusammensetzungen enthält, deren Vermischen zu einer wässrigen Zusammensetzung führt, wie sie in Anspruch 24 oder 25 definiert ist.

28. Verwendung einer Zusammensetzung, wie sie in einem der Ansprüche 1 bis 25 definiert ist, zum Entfärben und gleichzeitigen Färben.
